(19) Europäisches Patentamt
European Patent Office
Office européen des brevets

(11) EP 2 702 990 A1

(12) **EUROPEAN PATENT APPLICATION**
published in accordance with Art. 153(4) EPC

(43) Date of publication:
**05.03.2014 Bulletin 2014/10**

(21) Application number: **12776206.0**

(22) Date of filing: **27.04.2012**

(51) Int Cl.:
***A61K 31/215*** (2006.01) ***A01K 67/027*** (2006.01)
***A61K 31/231*** (2006.01) ***A61P 9/06*** (2006.01)
***G01N 33/15*** (2006.01) ***G01N 33/50*** (2006.01)

(86) International application number:
**PCT/JP2012/061314**

(87) International publication number:
**WO 2012/147902 (01.11.2012 Gazette 2012/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.04.2011 JP 2011099523**
**31.01.2012 JP 2012018977**

(71) Applicant: **Toho University**
**Ota-ku**
**Tokyo**
**143-8540 (JP)**

(72) Inventor: **SUGIYAMA, Atsushi**
**Tokyo 143-8540 (JP)**

(74) Representative: **Graf von Stosch, Andreas et al**
**Graf von Stosch**
**Patentanwaltsgesellschaft mbH**
**Prinzregentenstrasse 22**
**80538 München (DE)**

# (54) ANTIARRHYTHMIC DRUG, ATRIAL FIBRILLATION INHIBITOR, MODEL OF SUSTAINED ATRIAL FIBRILLATION AND METHOD FOR PRODUCING SAME, AND METHOD FOR SCREENING ATRIAL FIBRILLATION INHIBITOR

(57) An atrial fibrillation inhibitor, including: a compound expressed by one of the following Structural Formulas (I) to (VI) or a pharmacologically acceptable salt thereof, wherein the atrial fibrillation inhibitor inhibits atrial fibrillation:

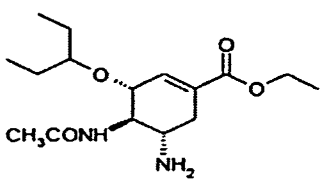

Structural Formula (I)

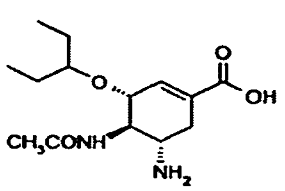

Structural Formula (II)

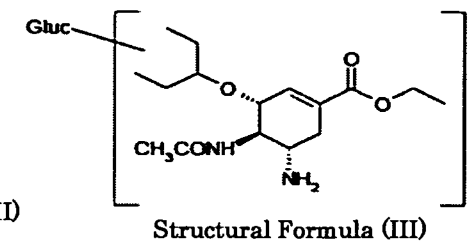

Structural Formula (III)

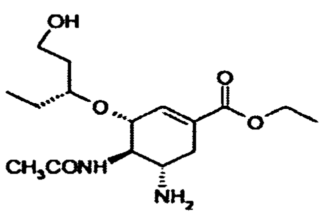

Structural Formula (IV)

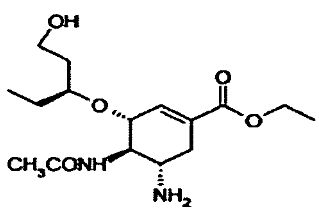

Structural Formula (V)

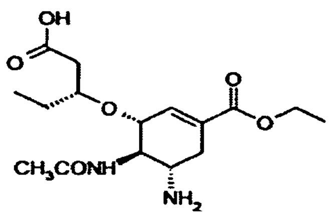

Structural Formula (VI)

where in the Structural Formula (III), Gluc refers to glucuronic acid.

EP 2 702 990 A1

# FIG. 5C

+ 5 min 50 sec (after the beginning of administration)

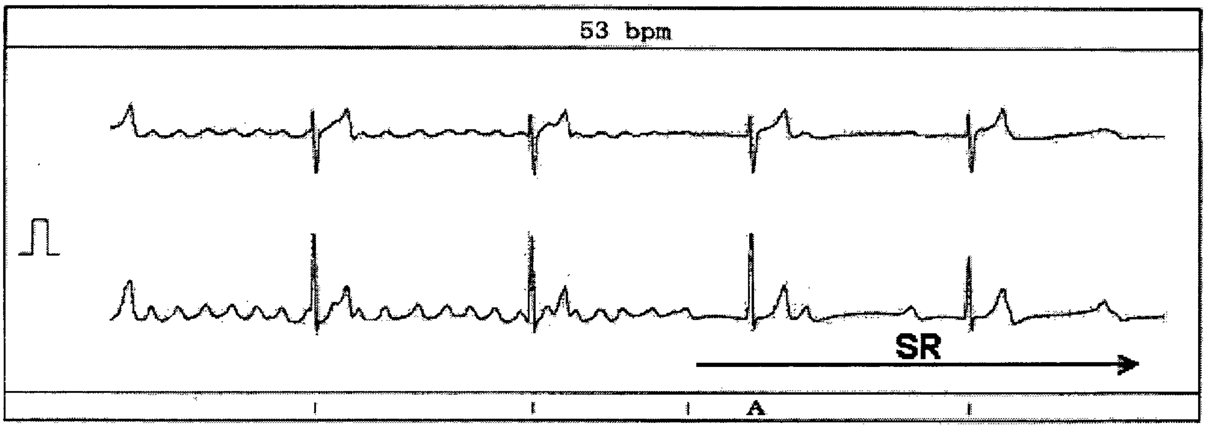

## Description

### Technical Field

The present invention relates to an antiarrhythmic drug, an atrial fibrillation, a model of sustained atrial fibrillation and a method for producing the model, and a method for screening for an atrial fibrillation inhibitor using the model of sustained atrial fibrillation.

### Background Art

Atrial fibrillation is one type of arrhythmia in which the pacemaker of the heart called the sinus node suffers from functional disorders, so that normal electric excitation of the atrium does not start and the muscle of the atrium shivers irregularly and finely at a rate of 300 times to 500 times per minute which is 5 or more times that in normal states, and as a result there is no contraction nor dilation of the atrium serving as a supplemental pump.
Atrial fibrillation is arrhythmia which clinically occurs most frequently, and develops more frequently with increasing age. In Japan, it is said that atrial fibrillation is developed in 2% to 4% of people in their sixties, 5% to 6% of people in their seventies, and 8% to 10% of people in their eighties.
Development of atrial fibrillation inhibits atrial contraction and prevents the ventricle from being sufficiently filled with blood at the diastolic phase. As a result, the heart's functions decrease to lead to reduction of the amount of blood supplied from the heart. The atrium enlarges to store blood temporarily when the ventricle becomes small as a result of contraction, and uses the stored blood for filling the ventricle with blood next time (i.e., a reservoir function). However, this function is impaired due to atrial fibrillation.
Furthermore, atrial fibrillation easily forms blood aggregates (thrombi) due to intra-atrial congestion, and the thrombi are released from the heart and cause thrombotic or embolic diseases of organs in the whole body (e.g., cerebral infarction).
Thus, although atrial fibrillation is not fatal arrhythmia, its complicating diseases are problematic.
Atrial fibrillation is broadly divided into two types: idiopathic atrial fibrillation and chronic atrial fibrillation. Examples of idiopathic atrial fibrillation include transient atrial fibrillation in which, even when atrial fibrillation occurs for some reason, atrial fibrillation no longer occurs by eliminating the cause; and paroxysmal atrial fibrillation in which atrial fibrillation is spontaneously terminated, but repeatedly occurs. Examples of chronic atrial fibrillation include sustained atrial fibrillation in which atrial fibrillation is sustained, but can be defibrillated and return to sinus rhythm by the action of drugs or electrical stimulation; and permanent atrial fibrillation in which atrial fibrillation is sustained, and cannot be defibrillated by the action of drugs or electrical stimulation.
Generally, atrial fibrillation progresses from transient atrial fibrillation, to paroxysmal atrial fibrillation, sustained atrial fibrillation and permanent atrial fibrillation in this order. At present, permanent atrial fibrillation cannot be treated. Therefore, it has been desired to prevent progression of atrial fibrillation at a stage of sustained atrial fibrillation.
Examples of a method for treating atrial fibrillation include an electrical therapy or a drug therapy.
The electrical therapy has rapid and high effects, but applies undue load on patients due to electric shock under general anesthesia. In addition, only the medical profession can subject patients to the electrical therapy because special equipment is required for the electrical therapy, which is problematic.
The drug therapy is advantageous in that patients themselves can participate in the therapy by taking drugs, which is simpler than the electrical therapy.
Warfarin, an antithrombotic drug, has been used as a preventive drug for atrial fibrillation. Meanwhile, as a therapeutic drug for atrial fibrillation, primarily used are Class Ia agents (e.g., disopyramide or cibenzoline) and Class Ic agents (e.g., flecainide or pilsicainide) according to the Vaughan Williams classification. For atrial fibrillation accompanied with hypertrophic cardiomyopathy, Class III agents (e.g., amiodarone, sotalol or nifekalant) have been used.
However, the above-listed drugs are problematic in causing adverse side effects. For example, disopyramide, which is a Class Ia agent, is problematic in that it has a negative inotropic effect, therefore, is unsuitable for patients of heart failure or left ventricular dysfunction, and that it may cause QRS and QT prolongations and proarrhlythmia. Flecainide and pilsicainide, which are Class Ic agents, are problematic in causing many adverse side effects on circulatory system. Amiodarone, which is a Class III agent, is problematic in causing adverse side effects on organs other than the heart such as the lungs, the thyroid gland, and the eyes.
These clinically used preventive drugs or therapeutic drugs for atrial fibrillation are not selected based on some test for evaluating effects on atrial fibrillation but empirically administered. Additionally, there is no test system for evaluating effects on atrial fibrillation, which is problematic.
It is desired to use a test system which simulates well clinical atrial fibrillation in an evaluation test for reevaluating efficacy of existing antiarrhythmic drugs against atrial fibrillation or in a screening for new atrial fibrillation inhibitors. Example of the test system includes a system using a model of atrial fibrillation reflecting clinical atrial fibrillation.

A model which applies reduced load on animals and which allows accurate evaluation is highly effective.

There has been proposed, as the model of atrial fibrillation, an aconitine model in which aconitine is topically administered to the auricle to thereby cause locally originated atrial fibrillation (see NPL 1).

However, the aconitine model is problematic in that it is not suitable for screening for atrial fibrillation inhibitors because it does not directly concerned with clinical atrial fibrillation.

Also, there has been proposed an abacterial pericarditis model in which atrial arrhythmia is allowed to be easily induced by abacterially scattering talc powder onto a surface of the atrial muscle to thereby cause pericarditis (see NPL 2). This model has been used for examining a developmental mechanism of atrial fibrillation.

However, the abacterial pericarditis model is problematic in that it is not suitable for screening for atrial fibrillation inhibitors effective for chronic atrial fibrillation because atrial fibrillation in the model results from pericarditis, so that it only reflects very limited atrial fibrillation which occurs after cardiac surgery.

There has been proposed a frequent stimulation-induced model in which atrial fibrillation is induced by frequent atrial stimulation in goats (see NPL 3).

However, this model is problematic in that it is not suitable for screening for atrial fibrillation inhibitors because goats are too big to use as experimental animals, and few institutions or researchers can handle goats all over the world.

Therefore, at present, keen demand has arisen for providing an antiarrhythmic drug or an atrial fibrillation inhibitor which has an excellent inhibitory effect on arrhythmia including atrial fibrillation and has high safety with reduced adverse side effects; a model of sustained atrial fibrillation which can be suitably used for screening for the atrial fibrillation inhibitor and a method for producing the model; and a method for screening for the atrial fibrillation inhibitor which allows efficient and simple screening for the atrial fibrillation inhibitor using the model of sustained atrial fibrillation.

Citation List

Patent Literature

NPL 1: Moe et al., 1959, Am. Heart. J. 58, 59-70

NPL 2: Page et al., 1986, J. Am Coll Cardiol., 8, 872-879

NPL 3: Maurits C.E.F. Wijffels et al., 1995, Circulation, 92, 1954-1968


Summary of Invention

Technical Problem

The present invention aims to solve the above existing problems and achieve the following object. An object of the present invention is to provide an antiarrhythmic drug or an atrial fibrillation inhibitor which has an excellent inhibitory effect on arrhythmia including atrial fibrillation and has high safety with reduced adverse side effects; a model of sustained atrial fibrillation which can be suitably used for screening for the atrial fibrillation inhibitor and a method for producing the model; and a method for screening for the atrial fibrillation inhibitor which allows efficient and simple screening for the atrial fibrillation inhibitor using the model of sustained atrial fibrillation.

Solution to Problem

The present inventors conducted extensive studies to solve the above problems and as a result have obtained the following findings.

That is, the present inventors have found that an atrial fibrillation inhibitor containing a compound expressed by one of the following Structural Formulas (I) to (VI) or a pharmacologically acceptable salt thereof and inhibiting atrial fibrillation has an excellent inhibitory effect on atrial fibrillation and has high safety with reduced adverse side effects. The present invention has been accomplished on the basis of this finding.

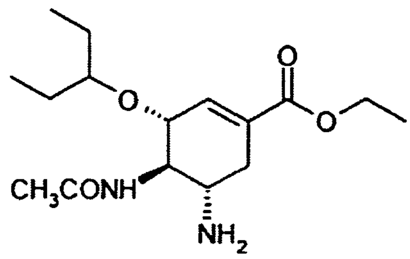

Structural Formula (I)

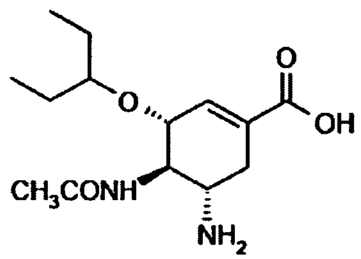

Structural Formula (II)

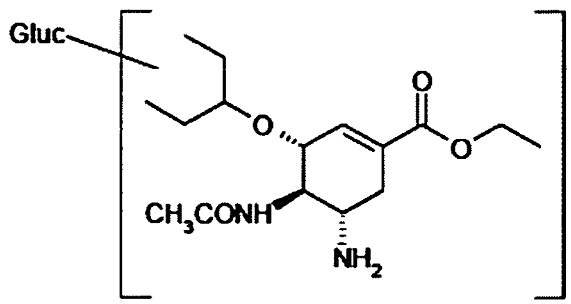

Structural Formula (III)

In the Structural Formula (III), Gluc refers to glucuronic acid.

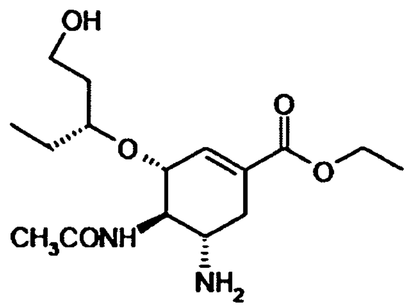

Structural Formula (IV)

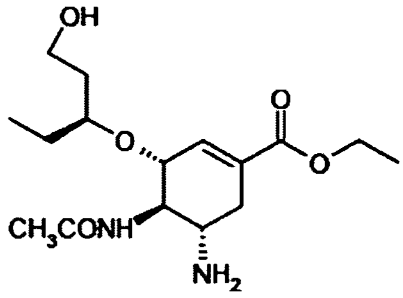

Structural Formula (V)

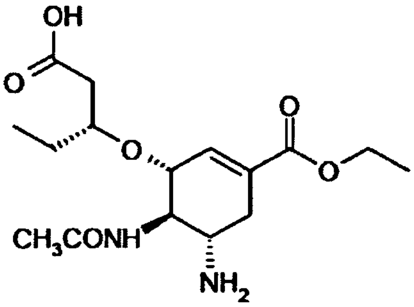

Structural Formula (VI)

The present invention is based on the above finding obtained by the present inventors, and means for solving the above problems are as follows.

<1> An atrial fibrillation inhibitor, including:

a compound expressed by one of the following Structural Formulas (I) to (VI) or a pharmacologically acceptable salt thereof,
wherein the atrial fibrillation inhibitor inhibits atrial fibrillation:

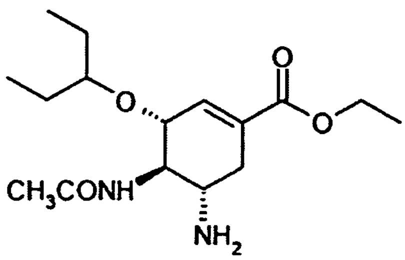

Structural Formula (I)

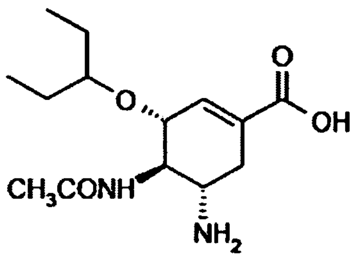

Structural Formula (II)

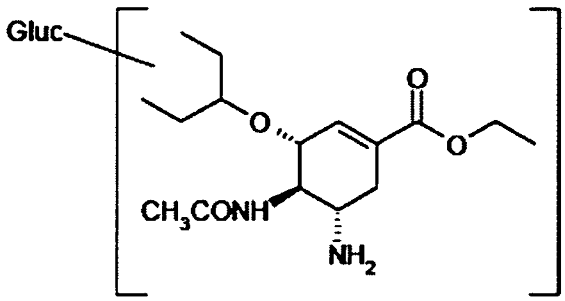

Structural Formula (III)

where in the Structural Formula (III), Gluc refers to glucuronic acid,

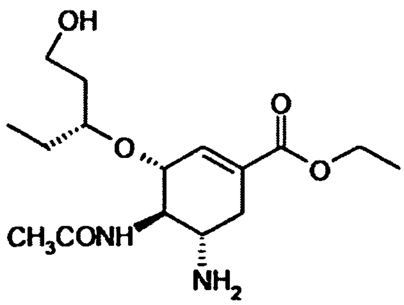

Structural Formula (IV)

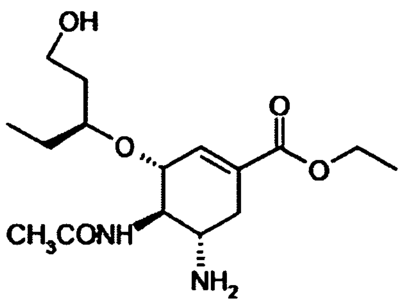

Structural Formula (V)

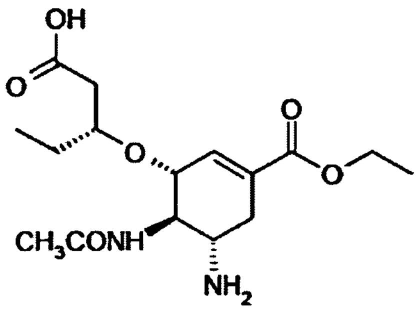

Structural Formula (VI).

<2> The atrial fibrillation inhibitor according to <1>, wherein the atrial fibrillation is idiopathic atrial fibrillation or chronic atrial fibrillation.

<3> A method for producing a model of sustained atrial fibrillation, including:

inserting an electrode catheter into a region of a dog containing an atrioventricular node and applying high-frequency current to the region from the electrode catheter to break the atrioventricular node and block atrioventricular conduction, to thereby dilate and enlarge the atrium thereof, and
pacing the atrium which has been dilated and enlarged.

<4> The method for producing a model of sustained atrial fibrillation according to <3>, wherein the pacing is pacing the atrium at 500 bpm to 700 bpm for 4 weeks or more.

<5> A model of sustained atrial fibrillation, wherein the model of sustained atrial fibrillation is produced by the method for producing a model of sustained atrial fibrillation according to <3> or <4>.

<6> The model of sustained atrial fibrillation according to <5>, wherein atrial fibrillation is sustained for at least one week.

<7> A method for screening for an atrial fibrillation inhibitor, including:

administering a test substance to the model of sustained atrial fibrillation according to <5> or <6>; and
screening for, as an atrial fibrillation inhibitor, the test substance that inhibits atrial fibrillation in the model of sustained atrial fibrillation after the administering.

<8> The method for screening for an atrial fibrillation inhibitor according to <7>, further including: pacing the atrium of the model of sustained atrial fibrillation in which atrial fibrillation has been inhibited in the screening, to thereby re-induce atrial fibrillation therein.

<9> The method for screening for an atrial fibrillation inhibitor according to <8>, wherein the pacing is pacing the atrium of the model of sustained atrial fibrillation at 500 bpm to 700 bpm for at least 1 week within 1 week after atrial fibrillation has been inhibited.

<10> An antiarrhythmic drug, including:

a compound expressed by one of the following Structural Formulas (I) to (VI) or a pharmacologically acceptable salt thereof,
wherein the antiarrhythmic drug inhibits arrhythmia:

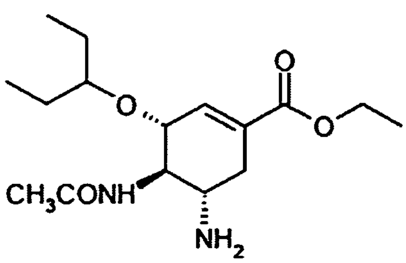

Structural Formula (I)

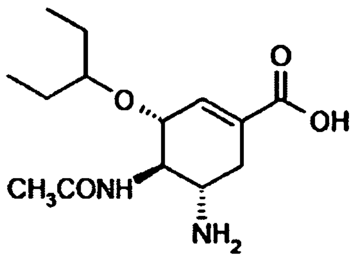

Structural Formula (II)

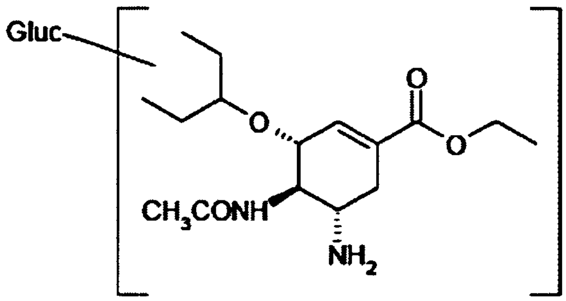

Structural Formula (III)

where in the Structural Formula (III), Gluc refers to glucuronic acid,

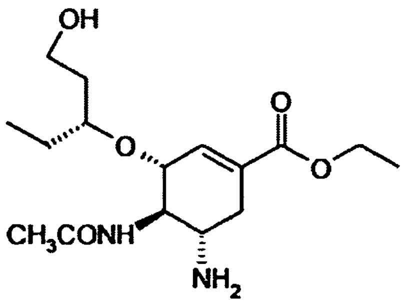

Structural Formula (IV)

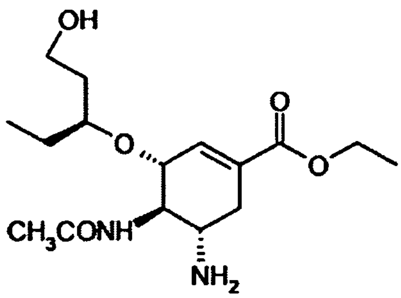

Structural Formula (V)

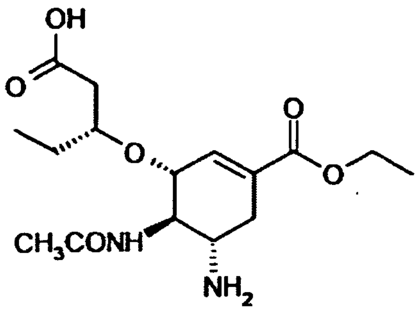

Structural Formula (VI).

Advantageous Effects of Invention

The present invention can provide an antiarrhythmic drug or an atrial fibrillation inhibitor which has an excellent inhibitory effect on arrhythmia including atrial fibrillation and has high safety with reduced adverse side effects; a model of sustained atrial fibrillation which can be suitably used for screening for the atrial fibrillation inhibitor and a method for producing the model; and a method for screening for the atrial fibrillation inhibitor which allows efficient and simple screening for the atrial fibrillation inhibitor using the model of sustained atrial fibrillation. These can solve the above existing problems and achieve the above object.

Brief Description of Drawings

Fig. 1A shows one exemplary MRI image of the heart in a normal beagle dog.
Fig. 1B shows one exemplary MRI image of the heart in a beagle dog which has been subjected to an atrium dilating and enlarging step (chronic atrioventricular block model).
Fig. 2A shows one exemplary echocardiogram of a normal beagle dog.
Fig. 2B shows one exemplary echocardiogram of a beagle dog which have been subjected to an atrium dilating and enlarging step (chronic atrioventricular block model).
Fig. 2C shows one exemplary echocardiogram of a beagle dog which have been subjected to an atrium dilating and enlarging step (chronic atrioventricular block model).
Fig. 3 shows one exemplary electrocardiogram at an atrium pacing step of a beagle dog of Production Example 1 (model of sustained atrial fibrillation).
Fig. 4 shows one exemplary electrocardiogram at an atrium pacing step of a beagle dog of Comparative Production Example 1.
Fig. 5A shows an electrocardiogram at - 1 min 33 sec before the beginning of administration of oseltamivir of an oseltamivir-administered group in Test Example 1.
Fig. 5B shows an electrocardiogram at + 2 min 49 sec after the beginning of administration of oseltamivir of an oseltamivir-administered group in Test Example 1.
Fig. 5C shows an electrocardiogram at + 5 min 50 sec after the beginning of administration of oseltamivir of an oseltamivir-administered group in Test Example 1.
Fig. 6A shows an electrocardiogram at + 6 min 00 sec to + 14 min 00 sec after the beginning of administration of disopyramide of a disopyramide-administered group in Test Example 1.
Fig. 6B shows an electrocardiogram at - 22 min 54 sec before the beginning of administration of disopyramide of a disopyramide-administered group in Test Example 1.
Fig. 6C shows an electrocardiogram at + 12 min 15 sec after the beginning of administration of disopyramide of a disopyramide-administered group in Test Example 1.
Fig. 7 shows results of an oseltamivir-administered group in Test Example 3.
Fig. 8 shows results of a pilsicainide-administered group in Test Example 3.
Fig. 9 shows results of a disopyramide-administered group in Test Example 3.
Fig. 10 shows results of a mexiletine-administered group in Test Example 3.

Description of Embodiments

(Atrial fibrillation inhibitor and antiarrhythmic drug)

An atrial fibrillation inhibitor of the present invention contains at least a compound expressed by one of the following Structural Formulas (I) to (VI) or a pharmacologically acceptable salt thereof, and, if necessary, further contains other ingredients.

An antiarrhythmic drug of the present invention contains at least a compound expressed by one of the following Structural Formulas (I) to (VI) or a pharmacologically acceptable salt thereof, and, if necessary, further contains other ingredients.

Next, both the atrial fibrillation inhibitor and the antiarrhythmic drug of the present invention will be described.

Among the above compounds, the above atrial fibrillation inhibitor or the above antiarrhythmic drug is preferably a compound that inhibits a sodium ion ($Na^+$) channel highly selective to atrial myocytes, particularly preferably a compound expressed by the following Structural Formula (I) or a pharmacologically acceptable salt thereof. Notably, the fact that the atrial fibrillation inhibitor or the antiarrhythmic drug has an inhibitory effect to atrial muscle $Na^+$ channel can be confirmed based on the width of P wave in an electrocardiogram which is an index reflecting the inhibitory effect to atrial muscle $Na^+$ channel.

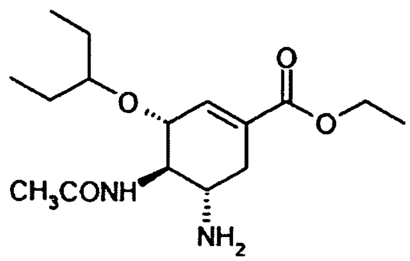

Structural Formula (I)

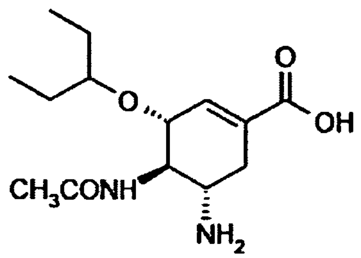

Structural Formula (II)

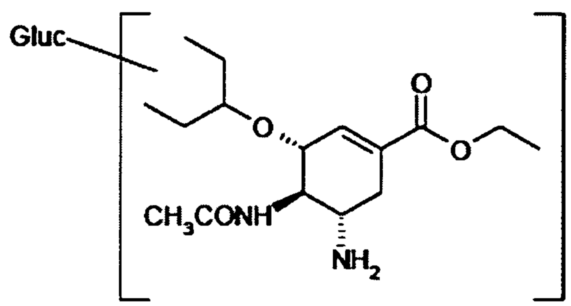

Structural Formula (III)

In the Structural Formula (III), Gluc refers to glucuronic acid. Also, the compound expressed by the Structural Formula (III) is a glucuronic acid conjugate of the Structural Formula (I).

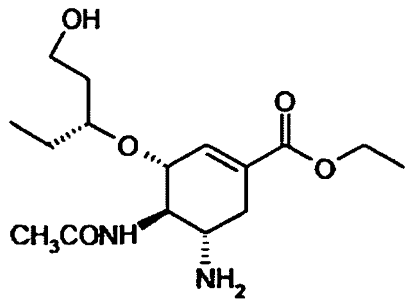

Structural Formula (IV)

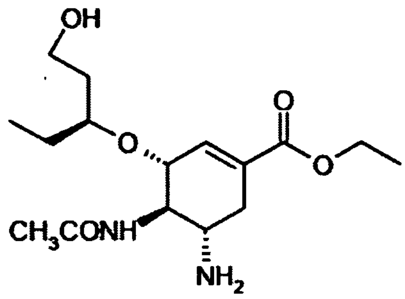

Structural Formula (V)

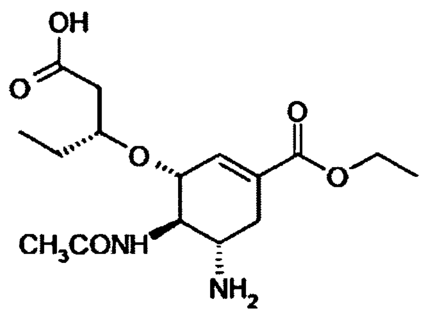

Structural Formula (VI)

The compound expressed by the Structural Formula (I) is a compound called oseltamivir known as a compound having anti-influenza virus activity, and its commercially available product is marketed under the trade name: TAMIFLU (registered trademark) (product of CHUGAI PHARMACEUTICAL CO., LTD.).

A method for confirming the structure and/or amount of the compound expressed by one of the above Structural Formulas (I) to (VI) or a pharmacologically acceptable salt thereof in the atrial fibrillation inhibitor or the antiarrhythmic drug is not particularly limited and may be appropriately selected depending on the intended purpose from various analysis methods. Examples thereof include elemental analysis, mass spectrometry, proton nuclear magnetic resonance analysis, $^{13}$C nuclear magnetic resonance analysis and infrared absorption analysis.

An amount of the compound expressed by one of the above Structural Formulas (I) to (VI) or a pharmacologically acceptable salt thereof in the atrial fibrillation inhibitor or the antiarrhythmic drug is not particularly limited and may be appropriately selected depending on the intended purpose. Also, the atrial fibrillation inhibitor or the antiarrhythmic drug may be the compound expressed by one of the above Structural Formulas (I) to (VI) or a pharmacologically acceptable salt thereof itself.

-Production method for the compound expressed by one of the above Structural Formulas (I) to (VI) or a pharmacologically acceptable salt thereof-

The compound expressed by one of the above Structural Formulas (I) to (VI) or a pharmacologically acceptable salt thereof may be a commercially available product or may be obtained through chemical synthesis.

The production method for the compound expressed by one of the above Structural Formulas (I) to (VI) or a pharmacologically acceptable salt thereof is not particularly limited and may be appropriately selected depending on the intended purpose. The synthesis method for the compound of formula 116 in Japanese Patent Application Laid-Open (JP-A) No. 2000-517306 can suitably be employed.

Specifically, the production method preferably contains one of the processes AA, AB, AC, AD, AE, AF, AG, AH, AI, AJ and AK in the following schemes 1 and 2 or a sequential combination thereof, or one of the processes AL, AM, AN, AO and AP in the following scheme 3 or a sequential combination thereof.

In the present invention, "sequential combination" means more than one process wherein the individual processes are performed one after the other in a desired order.

In addition, isolation, separation, purification, etc. may be performed, and a phase at which isolation, separation, purification, etc. are performed is not particularly limited and may be appropriately selected intended purpose. They may be performed before or after any one of the individual processes.

Notably, in the following schemes 1 to 3, "Et" refers to an ethyl group, "Ms" refers to a mesyl group, and "Ac" refers to an acetyl group.

Scheme 1

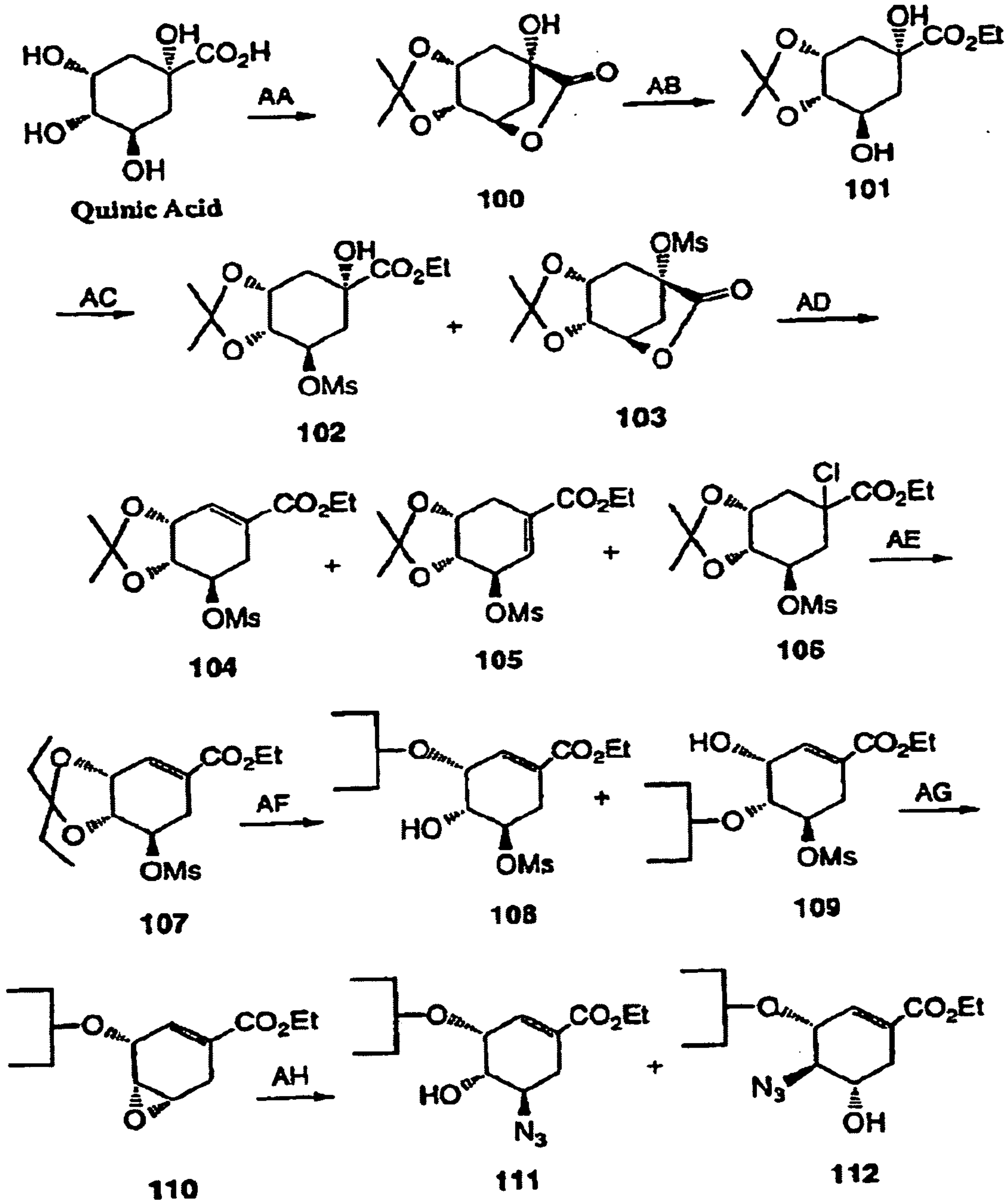


Quinic Acid 100 101

102 103

104 105 106

107 108 109

110 111 112

Scheme 2

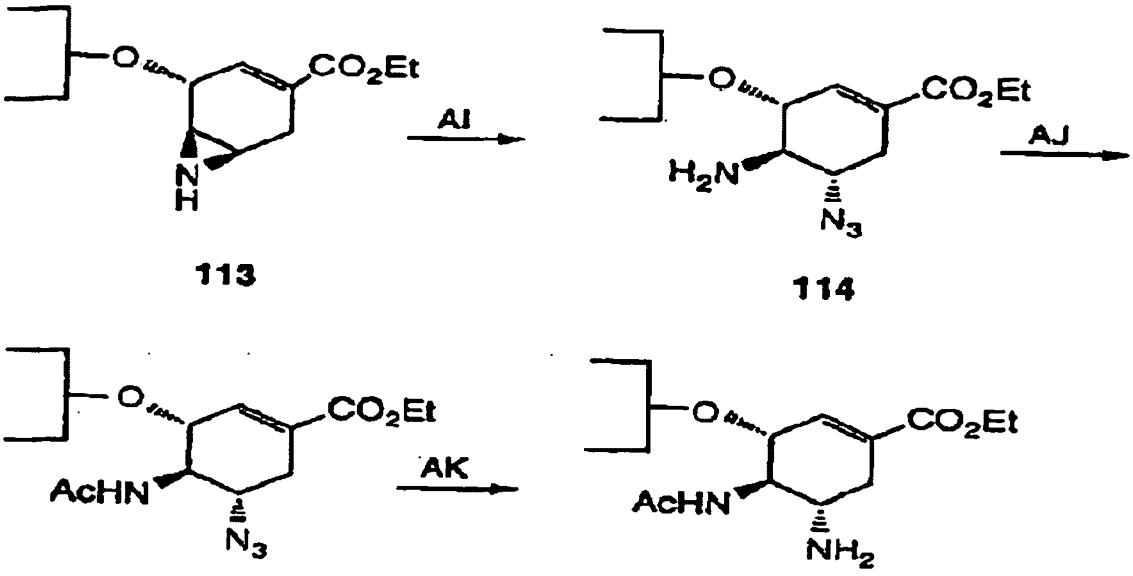


113

114

115

Structural Formula (I)

More specifically, a compound expressed by the Structural Formula (I) can be synthesized in the following manner: a compound of formula 110 is treated with sodium azide to form a compound of formula 111; the compound of formula 111 is treated with a reductive reagent to form a compound of formula 113; the compound of formula 113 is treated with sodium azide to form a compound of formula 114; the compound of formula 114 is treated with an acetylating reagent to form a compound of formula 115; and the compound of formula 115 is subjected to catalytic hydrogenation to obtain the compound expressed by the Structural Formula (I).

The following scheme 3 depicts the synthesis of compound 206 expressed by the Structural Formula (I) (R = $H_2$) by use of alternative nitrogen nucleophiles (March, "Advanced Organic Chemistry" 4th ed., pp. 425-427) to open epoxide 201. Oxidation of azidoalcohol 202 gives ketone 203 (Larock, "Comprehensive Organic Transformations", pp. 604-614) in which the β-axial NR group isomerizes to ketone 204 having the α-equatorial configuration. Reductive amination of the ketone 204 (Larock (*supra*), pp. 421 to 425) gives β-equatorial amine 205, which is acetylated to afford compound 206. Cleavage of the R moiety (Greene, "Protective Groups in Organic Synthesis", pp. 218-287) gives the compound expressed by the Structural Formula (I) (R = $H_2$).

<u>Scheme 3</u>

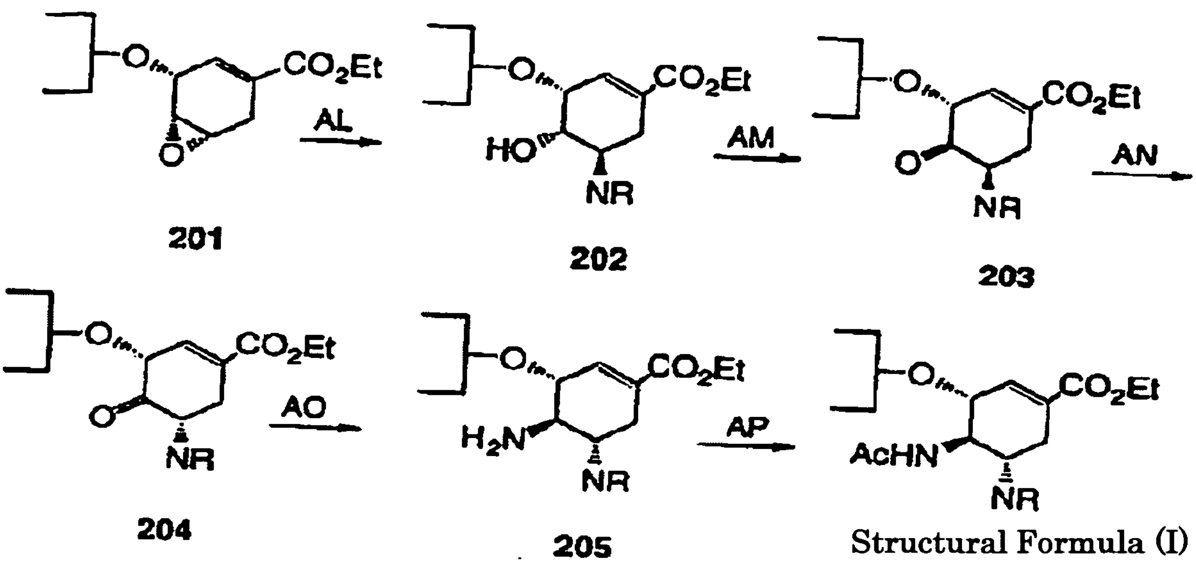


201

202

203

204

205

Structural Formula (I)

More specifically, in one exemplary method for synthesizing the compound expressed by the Structural Formula

(I), a compound of formula 201 is treated with an amine reagent to form a compound of formula 202; the compound of formula 202 is treated with an oxidation reagent to form a compound of formula 203; the compound of formula 203 is treated with a base to form a compound of formula 204; the compound of formula 204 is treated with a reductive amination reagent to form a compound of formula 205; and the compound of formula 205 is treated with an acetylating reagent to synthesize the compound expressed by the Structural Formula (I).

In each of the above schemes 1 to 3, it is preferred that to separate and/or purify desired reaction products or starting materials at each step or series of steps from one another.

A method for the separation and/or purification is not particularly limited and may be appropriately selected depending on the intended purpose. Examples thereof include multiphase extraction, crystallization from a solvent or solvent mixture, distillation, sublimation, and chromatography.

The chromatography is not particularly limited and may be appropriately selected depending on the intended purpose. Examples thereof include size exclusion chromatography, ion exchange chromatography, high pressure liquid chromatography, medium pressure liquid chromatography, low pressure liquid chromatography, small scale and preparative thin or thick layer chromatography, and techniques of small scale thin layer and flash chromatography.

The above separation and/or purification may be a treatment of a mixture with a reagent selected to bind to or render otherwise separable and/or purifiable a desired product, unreacted starting material, reaction by product, or the like.

Such reagents are not particularly limited and may be appropriately selected depending on the intended purpose. Examples thereof include adsorbents or absorbents such as activated carbon, molecular sieves and ion exchange media; acids preferably used in the case where a material to be separated and/or purified is a basic material; bases preferably used in the case where a material to be separated and/or purified is an acid material; binding reagents such as antibodies and binding proteins; selective chelators such as crown ethers; and liquid/liquid ion extraction reagents (LIX).

The above schemes 1 and 2 will now be described in more detail.

The compound of formula 100 (lactone) can be obtained in the following manner. Specifically, a solution of quinic acid (20 kg, 104 mol; [α]D-43.7° (c = 1.12, water); "Merck Index 11th ed.", 8071: [α]D-42° to -44° (water)), 2,2-dimethoxypropane and p-toluenesulfonic acid monohydrate in acetone is heated at reflux for two hours. The reaction is quenched by addition of sodium ethoxide in ethanol and most of the solvent is distilled in vacuo. The residue is partitioned between ethyl acetate and water. The aqueous layer is back-extracted with ethyl acetate and the combined organic layers are washed with aqueous sodium bicarbonate. Most of the ethyl acetate is distilled in vacuo to leave a pale yellow solid residue of the compound of formula 100.

The compound of formula 101 (hydroxy ester) can be obtained in the following manner. Specifically, a solution of the compound of formula 100 in absolute ethanol is treated with sodium ethoxide in ethanol. After two hours at room temperature, acetic acid is added and the solvent is distilled in vacuo. Ethyl acetate is added and the distillation continues to near dryness. The tan solid residue is obtained and dissolved in ethyl acetate at reflux, and hexane is added. Upon cooling, a white crystalline solid forms which is isolated by filtration to afford a mixture of the compound of formula 101 and the compound of formula 100.

The compoud of formula 102 (mesyl ester) can be obtained in the following manner. Specifically, a solution of the mixture of the compound of formula 101 and the compound of formula 100 suspended in dichloromethane is cooled to 0°C to 10°C and treated with methanesulfonyl chloride, followed by slow addition of triethylamine. An additional portion of methanesulfonyl chloride is added. After one hour, water and hydrochloric acid are added. The layers are separated and the organic layer is washed with water and then distilled in vacuo to leave a semi-solid residue which is a mixture of the compound of formula 102 and the compound of formula 103 (mesyl lactone). The residue is dissolved in ethyl acetate and cooled to -10° to -20°C for two hours. The compound of formula 103 is crystallized and separated by filtration and washed with cold ethyl acetate. The filtrate is concentrated to afford the compound of formula 102 as an orange resin.

The compound of formula 104 (mesyl acetonide) can be obtained in the following manner. Specifically, a solution of the compound of formula 102 and pyridine in dichloromethane is cooled to -20C° to -30°C and treated portionwise with sulfuryl chloride. After the exothermic reaction subsided, the resulting slurry is quenched with ethanol, warmed to 0°C, and washed successively with sulfuric acid, water and aqueous sodium bicarbonate. The obtained organic layer, which is a mixture of the compound of formula 104, the compound of formula 105 (allylic mesylate) and the compound of formula 106, is concentrated in vacuo and ethyl acetate is added. The compound of formula 105 is selectively removed by treatment of the ethyl acetate solution with pyrrolidine and tetrakis(triphenylphosphine)palladium(0) at ambient temperature for five hours, followed by washing with sulfuric acid. The organic layer is filtered through a pad of silica gel and eluted with ethyl acetate. The filtrate is concentrated in vacuo to leave a thick orange oil composed of a mixture of the compound of formula 104 and the compound of formula 106. The residue is dissolved in ethyl acetate at reflux and hexane is added. Upon cooling, he compound of formula 104 is crystallized and separated by filtration and washed with ethyl acetate in hexane. After drying in vacuo, the compound of formula 104 is obtained as pale yellow needles.

The compound of formula 107 (pentyl ketal) can be obtained in the following manner. Specifically, a solution of the compound of formula 104, 3-pentanone and perchloric acid i stirred for 18 hours. The volatiles are distilled in vacuo at ambient temperature and fresh 3-pentanone is added gradually as the distillation progresses. The reaction

mixture is filtered, toluene is added, and the resulting solution is washed successively with aqueous sodium bicarbonate, water and brine. Next, the organic layer is concentrated in vacuo and toluene is added gradually as the distillation progresses. At the time when no more distills, the residual orange oil is obtained as the compound of formula 107.

The compound of formula 108 (pentyl ether) can be obtained in the following manner. Specifically, a solution of the compound of formula 107 in dichloromethane is cooled to -30° to -20°C and treated with borane-methyl sulfide complex and trimethylsilyl trifluoromethanesulfonate. After one hour, aqueous sodium bicarbonate solution is slowly added. The mixture is warmed to ambient temperature and stirred for 12 hours. The obtained organic layer is filtered and concentrated in vacuo to leave a mixture of the compound of formula 108 and the compound of formula 109 as a gray waxy solid.

The compound of formula 110 (epoxide) can be obtained in the following manner. Specifically, a solution of the mixture of the compound of formula 108 and the compound of formula 109 in ethanol is treated with a solution of potassium hydrogen carbonate in water. After heating at 55C° to 65°C for two hours, the solution is cooled and twice extracted with hexanes. Unreacted compound of formula 109 remains in the aqueous ethanol layer. The combined hexane extracts were filtered and concentrated in vacuo to leave the compound of formula 110 as a flocculent white crystalline solid.

The compound of formula 111 (hydroxy azide) can be obtained in the following manner. Specifically, a mixture of the compound of formula 110, sodium azide and ammonium chloride in water and ethanol is heated at 70C° to 75°C for eight hours. Aqueous sodium bicarbonate is added and the ethanol is distilled in vacuo. The aqueous residue is extracted with ethyl acetate and the extract is washed with water. The water wash is back-extracted with ethyl acetate. The combined organic extracts are washed with brine, dried over anhydrous sodium sulfate, filtered and concentrated in vacuo to leave a mixture of the compound of formula 111 and the compound of formula 112 as a dark brown oil.

The compound of formula 113 (aziridine) can be obtained in the following manner. Specifically, the mixture of the compound of formula 111 and the compound of formula 112 is three times co-evaporated in vacuo from anhydrous acetonitrile and then dissolved in anhydrous acetonitrile. A solution of anhydrous triphenylphosphine in anhydrous tetrahydrofuran and anhydrous acetonitrile is added dropwise over two hours. The mixture is heated at reflux for six hours then concentrated in vacuo to leave a golden paste composed of the compound of formula 113, triphenylphosphine oxide and traces of triphenylphosphine. The paste is triturated with diethyl ether. Most of the insoluble triphenylphosphine oxide is removed by filtration and washed with diethyl ether. The filtrate is concentrated in vacuo to leave a dark brown oil which is dissolved in aqueous methanol and extracted three times with hexanes to remove triphenylphosphine. The hexane extracts are extracted with aqueous methanol and the combined aqueous methanol layers are concentrated in vacuo. The residue is twice co-evaporated in vacuo from anhydrous acetonitrile to leave a mixture of the compound of formula 113 and triphenylphosphine oxide as a dark brown oil.

The compound of formula 115 (acetamido azide) can be obtained in the following manner. Specifically, a mixture of the compound of formula 113, triphenylphosphine oxide, sodium azide and ammonium chloride in dimethylformamide is heated to 80C° to 85°C for five hours. Sodium bicarbonate and water are added thereto. The compound of formula 114 is isolated from the reaction mixture by six extractions with hexanes. The combined hexane extracts are concentrated in vacuo and dichloromethane is added. Aqueous sodium bicarbonate is added, followed by addition of acetic anhydride. After stirring for one hour at ambient temperature, the aqueous layer is discarded. The organic phases aree concentrated in vacuo and dissolved in ethyl acetate at reflux. Upon cooling, the compound of formula 115 is crystallized and isolated by filtration. After washing with cold ethyl acetate in hexane and drying in vacuo at ambient temperature, pure compound of formula 115 is obtained as off-white crystals.

The compound expressed by the Structural Formula (I) can be obtained in the following manner. Specifically, a mixture of the compound of formula 115 and Lindlar catalyst in absolute ethanol is stirred for 18 hours while hydrogen (1 atm) is bubbled through the mixture. Filtration through Celite and concentration of the filtratein in vacuo afford the compound expressed by the Structural Formula (I) as a foam. Notably, this foam will be solidified when left to stand.

A phosphoric acid salt of the compound expressed by the Structural Formula (I) can be obtained in the following manner. Specifically, a solution of the compound expressed by the Structural Formula (I) in acetone is refluxed and treated with phosphoric acid in absolute ethanol. Crystallization commences immediately and after cooling to 0°C for 12 hours the precipitate is collected by filtration to afford the phosphoric acid salt of the compound expressed by the Structural Formula (I) as colorless needles.

A hydrochloric acid salt of the compound expressed by the Structural Formula (I) can be obtained in the following manner. Specifically, an absolute ethanol solution of a solution of the compound expressed by the Structural Formula (I) in acetone is treated with hydrogen chloride in ethanol. Most of the ethanol is evaporated in vacuo and the oily residue is stirred with ethyl acetate until solid forms. Hexanes are gradually added to the stirred mixture. After one hour at ambient temperature, the solid is collected by filtration, washed with diethyl ether and dried in vacuo, to afford the hydrochloric acid salt of the compound expressed by the Structural Formula (I) as an off-white solid.

A production method for the compound expressed by one of the Structural Formulas (II) to (VI) or a pharmacologically acceptable salt thereof is not particularly limited and may be appropriately selected depending on the intended

purpose. Notably, the compound expressed by one of the Structural Formulas (II) to (VI) or a pharmacologically acceptable salt thereof is known to be a metabolite of the compound expressed by the Structural Formula (I) or a pharmacologically acceptable salt thereof (see TAMIFLU (registered trademark) Pharmaceutical Interview Form, December, 2005 (16th revised edition).

<Other ingredients>

The other ingredients in the atrial fibrillation inhibitor or the antiarrhythmic drug are not particularly limited and may be appropriately selected depending on the intended purpose so long as they are pharmacologically acceptable. Examples thereof include known additives, supplements and water.
The additives or supplements are not particularly limited and may be appropriately selected depending on the intended purpose. Examples thereof include a disinfectant, a preserving agent, a binding agent, a thickener, an adhesive agent, an integrating agent, a colorant, a stabilizer, a pH adjuster, a buffer, a tonicity agent, a solvent, an antioxidant, a UV rays-preventing agent, a preventing agent for precipitation of crystals, a defoaming agent, a property improving agent and an antiseptic agent. These may be used alone or in combination of two or more thereof.
The disinfectant is not particularly limited and may be appropriately selected depending on the intended purpose. Examples thereof include cationic surfactants such as benzalkonium chloride, benzethonium chloride and cetylpyridinium chloride.
The preserving agent is not particularly limited and may be appropriately selected depending on the intended purpose. Examples thereof include p-hydroxybenzoate esters, chlorobutanol and clesol.
The binding agent, thickener and adhesive agent are not particularly limited and may be appropriately selected depending on the intended purpose. Examples thereof include starch, dextrin, maltitol, cellulose, methyl cellulose, ethyl cellulose, carboxymethyl cellulose, hydroxyethyl cellulose, hydroxypropyol cellulose, hydroxypropyolmethyl cellulose, carboxymethyl starch, pullulan, sodium alginate, ammonium alginate, propylene glycol alginic acid esters, guar gum, locust bean gum, gum Arabic, xanthane gum, gelatin, casein, polyvinyl alcohol, polyethylene oxide, polyethylene glycol, ethylene/propylene block polymers, sodium polyacrylates and polyvinylpyrrolidone.
The integrating agent is not particularly limited and may be appropriately selected depending on the intended purpose. Examples of the integrating agent include water, ethanol, propanol, simple syrup, glucose liquid, starch liquid, gelatin liquid, carboxymethyl cellulose, hydroxypropyl cellulose, hydroxypropyl starch, methyl cellulose, ethyl cellulose, shellac, calcium phosphate, calcium stearate and polyvinylpyrrolidone.
The colorant is not particularly limited and may be appropriately selected depending on the intended purpose. Examples thereof include titanium oxide and iron oxide.
The stabilizer is not particularly limited and may be appropriately selected depending on the intended purpose. Examples thereof include tragacanth, gum Arabic, gelatin, sodium pyrosulfite, ethylenediaminetetraacetic acid (EDTA), thioglycolic acid and thiolactic acid.
The pH adjuster and the buffer are not particularly limited and may be appropriately selected depending on the intended purpose. Examples thereof include sodium citrate, sodium acetate and sodium phosphate.
The tonicity agent is not particularly limited and may be appropriately selected depending on the intended purpose. Examples thereof include sodium chloride and glucose.
The amount of the other ingredients in the atrial fibrillation inhibitor or the antiarrhythmic drug is not particularly limited and may be appropriately selected depending on the intended purpose so long as the effects of the present invention are not impaired.

<Combinational use>

The atrial fibrillation inhibitor or the antiarrhythmic drug may be used alone or in combination with other drugs containing other active ingredients. The atrial fibrillation inhibitor or the antiarrhythmic drug also may be incorporated before use into another drug containing other active ingredients.
The other drugs containing other active ingredients are not particularly limited and may be appropriately selected depending on the intended purpose. Examples thereof include drugs clinically used as preventive drugs or therapeutic drugs for atrial fibrillation. Specific examples include disopyramide, aprindine, cibenzoline and pilsicainide. These may be used alone or in combination of two or more thereof. Since atrial fibrillation is difficult to relapse after termination of atrial fibrillation, the atrial fibrillation inhibitor or the antiarrhythmic drug is preferably used in combination with the above other drugs, and is particularly preferably used in combination with pilsicainide.

<Dosage form>

The dosage form of the atrial fibrillation inhibitor or the antiarrhythmic drug is not particularly limited and may

be appropriately selected depending on the intended purpose. Examples thereof include a solid preparation, a semi-solid preparation and a liquid preparation.

The atrial fibrillation inhibitor or the antiarrhythmic drug may be produced by a known method depending on the above dosage form thereof.

-Solid preparation-

The solid preparation is not particularly limited and may be appropriately selected depending on the intended purpose. Examples thereof include tablets, chewable tablets, foaming tablets, orally-disintegrating tablets, troches, drops, hard capsules, soft capsules, granules, powder, pills, dry syrups and infusions, suppositories, cataplasms and plasters.

-Semi-solid preparation-

The semi-solid preparation is not particularly limited and may be appropriately selected depending on the intended purpose. Examples thereof include electuaries, chewing gums, whip, jelly, ointments, creams, mousse, inhaler and nasal gel.

-Liquid preparation-

The liquid preparation is not particularly limited and may be appropriately selected depending on the intended purpose. Examples thereof include injections, syrups, drinks, suspensions, spirits, eye drops, aerosol and sprays, with injections being particularly preferred.

The production method of the atrial fibrillation inhibitor or the antiarrhythmic drug is not particularly limited and may be appropriately selected from known methods depending on, for example, the dosage form thereof.

<Administration>

The administration method, administration dose, administration period and administration target of the atrial fibrillation inhibitor or the antiarrhythmic drug are not particularly limited and may be appropriately selected depending on the intended purpose.

Examples of the administration method include an oral administration method, a parenteral administration method, a local administration method and an enteral administration method, with a parenteral administration method being preferred, with an administration method by injection being preferred.

The administration dose may be appropriately selected considering various factors of an administration target, such as the age, body weight, constitution, symptom and the presence or absence of administration of a drug containing other active ingredients. The total administration dose per day of the compound expressed by one of the Structural Formulas (I) to (IV) or a pharmacologically acceptable salt thereof serving as an active ingredient is preferably 3 mg/kg or more, more preferably 10 mg/kg or more, still more preferably 20 mg/kg or more, yet more preferably 25 mg/kg or more, particularly preferably 30 mg/kg or more.

Also, the upper limit of the administration dose is not particularly limited and may be appropriately selected depending on the intended purpose, but is preferably 2,000 mg/kg or less, more preferably 1,000 mg/kg or less, particularly preferably 100 mg/kg or less.

The above lower limits and upper limits of the administration dose may be appropriately combined, and the administration dose when they are combined is preferably 3 mg/kg to 2,000 mg/kg, more preferably 10 mg/kg to 1,000 mg/kg, still more preferably 20 mg/kg to 1,000 mg/kg, yet more preferably 25 mg/kg to 1,000 mg/kg, particularly preferably 30 mg/kg to 100 mg/kg.

The number at which the atrial fibrillation inhibitor or the antiarrhythmic drug is to be administered is not particularly limited and may be appropriately selected depending on the intended purpose. The atrial fibrillation inhibitor or the antiarrhythmic drug may be administered once a day or several times a day in a divided manner.

The animal species serving as the administration target is not particularly limited and may be appropriately selected depending on the intended purpose. Examples thereof include human, monkey, pig, bovine, sheep, goat, dog, cat, mouse, rat and bird, with human being suitably used.

<Applications>

The atrial fibrillation inhibitor or the antiarrhythmic drug of the present invention has excellent arrhythmia inhibitory effects, especially excellent inhibitory effects to atrial fibrillation, and has high safety with reduced adverse side

effects. Thus, the atrial fibrillation inhibitor or the antiarrhythmic drug can suitably be used as a preventive drug or a therapeutic drug for arrhythmia including atrial fibrillation. In addition, it is advantageous that the atrial fibrillation inhibitory effects are also effective to any of idiopathic atrial fibrillation (paroxysmal atrial fibrillation) and sustained atrial fibrillation. Here, in the present invention, the atrial fibrillation inhibitory effects may be effects of inhibiting at least atrial fibrillation and include defibrillation. The defibrillation means terminating atrial fibrillation to return it to sinus rhythm.

The atrial fibrillation inhibitor is effective to sustained atrial fibrillation and thus can prevent progression to permanent atrial fibrillation. Furthermore, the atrial fibrillation inhibitor can suitably be used as a preventive drug for complicating diseases such as thrombotic or embolic diseases of organs in the whole body (e.g., cerebral infarction).

(Model of sustained atrial fibrillation and method for producing the model)

A method of the present invention for producing a model of sustained atrial fibrillation includes: an atrium dilating and enlarging step and an atrium pacing step; and, if necessary, further includes other steps.

A model of sustained atrial fibrillation of the present invention is a model produced by the method of the present invention for producing a model of sustained atrial fibrillation.

Next, both the method for producing a model of sustained atrial fibrillation and the model of sustained atrial fibrillation will be described in detail.

<Atrium dilating and enlarging step>

The atrium dilating and enlarging step is a step of inserting an electrode catheter into a region of a dog containing an atrioventricular node and applying high-frequency current to the region from the electrode catheter to break the atrioventricular node and block atrioventricular conduction, to thereby dilate and enlarge the atrium thereof. The atrium dilating and enlarging step can be performed by the method described in Japanese Patent Application Laid-Open (JP-A) No. 2006-67950.

In the present invention, "dilation", "dilating" and "dilate" of the atrium the atrium mean that the atrial wall becomes thick, and "enlargement" "enlarging" and "enlarge" of the atrium mean that the volume of an inner space surrounded by the atrial wall increases. Thus, the dog which has undergone the above atrium dilating and enlarging step has a thickened atrium wall and an increased volume of the inner space of the atrium.

The type, size, age, sex, etc. of the dog used in the method for producing a model of sustained atrial fibrillation are not particularly limited and may be appropriately selected depending on the intended purpose. A beagle dog, which has successfully been used as an experimental animal, is preferred in terms of easiness in handing, and a one- to two-year-old beagle dog having a body weight of about 10 kg is more preferred.

When the atrium dilating and enlarging step is performed using the above dog, it is preferred that the dog is anesthetized before the atrium dilating and enlarging step.

The type of an anesthetic drug is not particularly limited and may be appropriately selected from known anesthetic drugs depending on the intended purpose. Examples thereof include pentobarbital and halothane. These may be used alone or in combination of two or more thereof.

A dose of the anesthetic drug to be administered is not particularly limited and may be appropriately selected depending on, for example, the type, size, age, sex, etc. of a dog to be used.

When the atrium dilating and enlarging step is performed using the above dog, it is preferred that the dog is anesthetized, followed by intubation to thereby supply a certain amount of oxygen or air from an oxygen or air supplying unit such as a ventilator for the purpose of providing stable respiration.

An amount of the oxygen or air to be supplied is not particularly limited and may be appropriately selected depending on, for example, a body weight of a dog to be used, but is preferably 15 mL/kg to 25 mL/kg.

The material, shape, diameter, length, etc. of the electrode catheter are not particularly limited and may be appropriately selected from known electrode catheters depending on the intended purpose. However, a catheter to a tip of which an electrode (herein may be referred to as "tip electrode") is attached is preferred. The catheter to a tip of which an electrode is attached may be a commercial product. Example thereof includes D7-DL-252 (catalog number, tip electrode: 4 mm, product of Cordis Webster Inc.).

A method for inserting the electrode catheter into the region containing the atrioventricular node of the dog is not particularly limited and may be appropriately selected depending on the intended purpose. However, the electrode catheter is preferably inserted through the femoral vein into the region containing the atrioventricular node. More preferably, the tip electrode of the electrode catheter is secured to a predetermined position.

It is preferred that, after determining a position at which the largest His bundle electrocardiogram (AV) can be recorded, the tip electrode of the electrode catheter is secured to a region at which a ratio of A wave indicating an atrial electrical excitation to V wave indicating a ventricular electrical excitation (A wave:V wave) is 2:1 or more. Example of such region includes a region containing the atrioventricular node.

A frequency at which current is applied to the region containing the atrioventricular node is not particularly limited and may be appropriately selected depending on the intended purpose, as long as it is a high-frequency. However, it is preferably a frequency which is sufficient to cauterize the intended region, more preferably 450 kHz to 550 kHz, particularly preferably 500 kHz. When the frequency is less than 450 kHz, the atrioventricular node may not be broken in some cases. When the frequency is more than 550 kHz, the atrioventricular node may be excessively cauterized to thereby break an undesired region.
The electric power to be applied to the region containing the atrioventricular node is not particularly limited and may be appropriately selected depending on the intended purpose, but is preferably 18 W to 22 W, more preferably 20 W. When it is less than 18 W, the atrioventricular node may not be broken in some cases. When the electric power is more than 22 W, the atrioventricular node may be excessively cauterized to thereby break an undesired region.
The period during which current is applied is not particularly limited and may be appropriately selected depending on the intended purpose.
After breaking the atrioventricular node of the dog, preferably 4 weeks or more, more preferably 4 weeks to 6 weeks are needed to dilate and enlarge the atrium thereof.
The atrium can be confirmed to be dilated and enlarged using, for example, magnetic resonance imaging (MRI), echocardiography, radiography, pressure application, or volume load measurement.
The atrium dilating and enlarging step results in a model of chronic atrioventricular block in which atrioventricular conduction is completely blocked. In the model of chronic atrioventricular block, the sinus node does not function as a pacemaker, so that the right ventricle and the left ventricle pump blood under the control of His bundle. Therefore, a function of pumping blood from the heart is deteriorated and heart rate is greatly reduced, which applies load on the heart. As a result, the heart is increased in weight about two-fold.

<Atrium pacing step>

The atrium pacing step is a step of pacing the atrium which has been dilated and enlarged in the atrium dilating and enlarging step.
In the present invention, "pacing" means an event which artificially generates electrical excitation in myocardial cells through electrical stimulation and propagates the electrical excitation to thereby cause cardiac contraction.
A method for pacing is not particularly limited and may be appropriately selected depending on the intended purpose, as long as it is a method which allows for pacing the dilated and enlarged atrium. Example thereof includes a method in which a pacemaker equipped with a pacing lead is implanted at a predetermined position.
The material, shape, size, etc. of the pacing lead and the pacemaker are not particularly limited and may be appropriately selected from known pacemakers depending on the intended purpose. Example of a commercially available pacing lead includes a pacing lead (product of OSCOR Inc.). Example of a commercially available pacemaker includes TNT-002 (catalog number, product of Taisho Biomed Instruments Co., Ltd.).
A position at which the pacing lead is implanted is not particularly limited and may be appropriately selected depending on the intended purpose, as long as it is a position which allows for pacing the dilated and enlarged atrium. However, the region containing the Bachmann bundle in the left atrium is preferred in terms of high-production efficiency of the model of sustained atrial fibrillation.
A position at which the pacemaker is implanted is not particularly limited and may be appropriately selected depending on the intended purpose. Example thereof includes a subcutaneous region of the buttocks.
After implanting the pacemaker, an interval of preferably 4 weeks or more, more preferably 4 weeks to 6 weeks are needed until vibration is applied to the atrium for the purpose of reducing load on animals.
The pacing speed is not particularly limited and may be appropriately selected depending on properties of individuals of interest, but is preferably 500 bpm to 700 bpm, more preferably 550 bpm to 650 bpm, particularly preferably 600 bpm. When the pacing speed is less than 500 bpm, the model of sustained atrial fibrillation cannot be produced in some cases. When the pacing speed is more than 700 bpm, permanent atrial fibrillation may be developed.
The pacing period is not particularly limited and may be appropriately selected depending on properties of individuals of interest, but is preferably 4 weeks or more, more preferably 6 weeks or more, particularly preferably 6 weeks to 12 weeks. When the pacing period is less than 4 weeks, the model of sustained atrial fibrillation cannot be produced in some cases. When the pacing period is more than 12 weeks, permanent atrial fibrillation may be developed.
A method for confirming whether the model of sustained atrial fibrillation is produced, that is, whether atrial fibrillation is sustained is not particularly limited and may be appropriately selected depending on the intended purpose. Example thereof includes a method for confirming by electrocardiography.
Notably, in the present invention, "atrial fibrillation is sustained" means that the atrium continues to irregularly vibrate at 300 bpm or more even after the completion of the atrium pacing step, that is, even after the pacing is terminated.

<Model of sustained atrial fibrillation>

The model of sustained atrial fibrillation in the present invention is a model in which the atrium has been dilated and enlarged, and atrial fibrillation is sustained. Therefore, the model is susceptible to thrombus formation. As a result, the model is advantageous in simulating well human clinical sustained atrial fibrillation.

The period during which atrial fibrillation is sustained in the model of sustained atrial fibrillation is not particularly limited and may be appropriately selected depending on the intended purpose, but is preferably 1 min or more, more preferably 1 hour or more, further preferably 24 hours or more, particularly preferably 1 week or more.

Notably, the model of sustained atrial fibrillation can be reproduced by subjecting the model of sustained atrial fibrillation in which atrial fibrillation has been inhibited or defibrillated to the atrium pacing step to thereby cause atrial fibrillation again.

<Applications>

The model of sustained atrial fibrillation of the present invention is or the method of the present invention for producing the model produces a model which simulates well clinical sustained atrial fibrillation, which allows atrial fibrillation to be sustained, and in which atrial fibrillation can be inhibited or defibrillated by using drugs. Therefore, the model can be suitably used for screening for atrial fibrillation inhibitors and re-evaluating efficacy of existing antiarrhythmic drugs. Also, the model of sustained atrial fibrillation can be suitably used for examining pathology such as a developmental mechanism of atrial fibrillation.

(Atrial fibrillation inhibitor screening method)

A method for screening for an atrial fibrillation inhibitor of the present invention includes an administration step and a screening step; and, if necessary, further includes other steps such as an atrial fibrillation re-inducing step.

<Administration step>

The administration step is a step of administering a test substance to the model of sustained atrial fibrillation of the present invention.

The test substance is not particularly limited and may be appropriately selected depending on the intended purpose. Examples thereof include compounds, nucleic acids, and proteins. Clinically used preventive drugs or therapeutic drugs for atrial fibrillation, newly developed drugs, and known medicines or drugs can also be used.

A method for administering the test substance is not particularly limited and may be appropriately selected depending on the type or form of the test substance. Examples thereof include an oral administration method, a parenteral administration method, a topical application method, or an enteral administration method.

An administration dose of the test substance is not particularly limited and may be appropriately selected depending on the age, body weight, or constitution of an administration target.

<Screening step>

The screening step is a step of screening for, as an atrial fibrillation inhibitor, the test substance that inhibits atrial fibrillation in the model of sustained atrial fibrillation after the administration step.

A method for determining whether atrial fibrillation is inhibited is not particularly limited and may be appropriately selected depending on the intended purpose. However, preferred is a method in which a change in an electrocardiogram, in particular, P wave between before and after administering the test substance is determined. More preferred is a method in which a change in an electrocardiogram, in particular, P wave is continuously observed before, during, and after administering the test substance.

A method for monitoring the electrocardiogram is not particularly limited. For example, known devices (e.g., long-term ECG analyzing system, trade name: HS1000 system, product of FUKUDA M-E KOGYO Co., LTD.) can be used.

In the model of sustained atrial fibrillation, atrial fibrillation is sustained, so that the electrocardiogram before administering the test substance shows an irregular P wave pattern corresponding to atrial fibrillation.

In the case where a P wave pattern on the electrocardiogram after administering the test substance is returning to or has returned to sinus rhythm, the test substance can be determined as an atrial fibrillation inhibitory substance and screened as an atrial fibrillation inhibitor.

Meanwhile, in the case where a P wave pattern on the electrocardiogram after administering the test substance does not change (atrial fibrillation is sustained) or becomes more irregular and/or finer (atrial fibrillation is facilitated), the test substance can be determined as an atrial fibrillation non-inhibitory substance.

Notably, the substance which causes an adverse side effect such as ventricular arrhythmia is not suitable for clinical application. Therefore, the above substance is preferably excluded from a test for clinical application even when it is screened as an atrial fibrillation inhibitor.

<Atrial fibrillation re-inducing step>

The atrial fibrillation re-inducing step is a step of pacing the atrium of the model of sustained atrial fibrillation in which atrial fibrillation has been inhibited in the screening step, to thereby re-induce atrial fibrillation therein. The atrial fibrillation re-inducing step is preferable in that one individual of the model of sustained atrial fibrillation can be repeatedly used for screening.

Also, it is also advantageous in simple procedure because atrial fibrillation can be re-induced without the atrium dilating and enlarging step in the method for producing the model of sustained atrial fibrillation.

At the atrial fibrillation re-inducing step, a method for pacing the atrium in the model of sustained atrial fibrillation in which atrial fibrillation has been inhibited may be the same as the methods used in the atrium pacing step.

At the atrial fibrillation re-inducing step, a pacing speed for pacing the atrium in the model of sustained atrial fibrillation in which atrial fibrillation has been inhibited is not particularly limited and may be appropriately selected depending on properties of individuals of interest, but is preferably 500 bpm to 700 bpm, more preferably 550 bpm to 650 bpm, particularly preferably 600 bpm. When the pacing speed is less than 500 bpm, atrial fibrillation cannot be re-induced in some cases.

The timing for performing the atrial fibrillation re-inducing step is not particularly limited and may be appropriately selected depending on the intended purpose. However, the atrial fibrillation re-inducing step is preferably performed within 1 week after atrial fibrillation is inhibited (atrial fibrillation returns to sinus rhythm). When the atrial fibrillation re-inducing step is preferably performed more than 1 week after atrial fibrillation has been inhibited, atrial fibrillation cannot be re-induced in some cases.

An individual in which atrial fibrillation has been re-induced at the atrial fibrillation re-inducing step can be subjected to the administration step and the screening step again, which is advantageous in that one individual of the model of sustained atrial fibrillation can be repeatedly used for screening.

Notably, in the case where atrial fibrillation cannot be re-induced at the atrial fibrillation re-inducing step, the model of sustained atrial fibrillation can be reproduced by subjecting the model of sustained atrial fibrillation in which atrial fibrillation has been inhibited to the method which is the same as the method for producing the model of sustained atrial fibrillation, and further the atrium dilating and enlarging step and the atrium pacing step.

<Applications>

The method for screening for atrial fibrillation inhibitors of the present invention is excellent as an in vivo evaluation system of atrial fibrillation because a canine model of sustained atrial fibrillation is used which has a similar developmental mechanism to human atrial fibrillation. In addition, the method can effectively and efficiently used for screening for new atrial fibrillation inhibitors used for preventing or treating atrial fibrillation; or re-evaluating existing drugs clinically used as therapeutic drugs for atrial fibrillation for a long period of time such as pilsicainide or disopyramide.

Notably, the method for screening for an atrial fibrillation inhibitor of the present invention can be suitably used as a method for screening for an antiarrhythmic drug.

Additionally, the method can be suitably used for a model for evaluating drug-induced exacerbation of arrhythmia; or observation of changes in heart rate, blood pressure, or an electrocardiogram after administration of a drug.

Examples

The present invention now will be specifically explained with reference to Examples, but the present invention is not limited thereto.

(Production Example 1)

-Atrium dilating and enlarging step-

Beagle dogs (n=6, about one year old, body weight: about 10 kg, female, KITAYAMA LABES CO., LTD.) were intravenously injected with 30 mg/kg of pentobarbital (product of Mitsubishi Tanabe Pharma Corporation), and simultaneously artificially ventilated by inserting a ventilator (SN-480-3, product of SHINANO manufacturing CO., LTD.) into the trachea to thereby supply 20 mL/kg of oxygen.

The femur of each of the beagle dogs was shaved, and disinfected with alcohol cotton. Thereafter, a guide wire

was inserted into the femoral vein, and then an electrode catheter to a tip of which a pacing electrode was attached (catalog number: D7-DL-252, tip electrode: 4 mm, product of Cordis Webster Inc.) was inserted through the femoral vein into the right ventricle. Then, an electrode for Holter electrocardiograph (long-term ECG analyzing system, trade name: HS1000 system, product of FUKUDA M-E KOGYO Co., LTD.) was mounted according to standard limb II leads. After determining a position at which the largest His bundle electrocardiogram (AV) can be recorded, the tip electrode was sewn on and secured to a region at which a ratio of A wave indicating an atrial electrical excitation to V wave indicating a ventricular electrical excitation (A wave:V wave) is 2:1 or more. Then, current was applied from the tip electrode of the electrode catheter to the region containing the atrioventricular node at 500 kHz and 20 W for 10 sec. Thus, the atrioventricular node was broken and the atrioventricular conduction was blocked. Thereafter, care was taken for the dog for 4 weeks.

--Confirmation of dilating and enlarging of atrium--

After 4 week-care, the atrium had been dilated and enlarged, which was confirmed by magnetic resonance imaging (MRI) and cardiac ultrasound (echocardiography). The MRI was performed by SIGNA PROFILE (product of General Electric Company, Milwaukee, WI). The echocardiography was performed by SSA-400 (product of TOSHIBA MEDICAL SYSTEMS CORPORATION).

Fig. 1A shows an MRI image of the heart in a normal beagle dog. Fig. 1B shows an MRI image of the heart in a beagle dog which has been subjected to the atrium dilating and enlarging step. Fig. 2A shows an echocardiogram of a normal beagle dog. Figs. 2B and 2C show echocardiograms of beagle dogs which have been subjected to the atrium dilating and enlarging step.

In Figs. 1A and 1B, and 2A to 2C, RA denotes the right atrium, RV denotes the right ventricle, LA denotes the left atrium, LV denotes the left ventricle, Ao denotes the aorta, PV denotes the pulmonary vein, and IVC denotes the descending aorta.

As can be seen from Figs. 1A and 1B, and 2A to 2C, the left atrium (LA) had been dilated and enlarged by the atrium dilating and enlarging step. Further, mitral regurgitation jet (MR jet) was observed in Fig. 2C. Thus, a model of chronic atrioventricular block was confirmed to be produced.

-Atrium pacing step-

Each of the beagle dogs which had been subjected to the atrium dilating and enlarging step was intravenously injected with 30 mg/kg of pentobarbital (product of Mitsubishi Tanabe Pharma Corporation), and simultaneously artificially ventilated by inserting a ventilator into the trachea to thereby supply 20 mL/kg of oxygen.

The buttocks of each of the beagle dogs were shaved, and disinfected with alcohol cotton. Thereafter, a pacemaker (catalog number: TNT-002, product of Taisho Biomed Instruments Co., Ltd.) connected with a pacing lead (product of OSCOR Inc.) was implanted in the buttocks. The pacing lead was traveled through a subcutaneous tunnel to the breast, where a tip electrode was sewn on and secured to the Bachmann bundle region in the left atrium which had been exposed by a thoracotomy. After implanting the pacemaker and the pacing lead, care was taken for the dog for 4 weeks.

Then, the pacemaker was operated at 600 bpm to thereby apply a vibration to the atrium for 6 weeks. In the case of operating the pacemaker at 600 bpm, a ratio of an electrical stimulation applied by the pacemaker (A) to an atrial muscle contraction reaction (B) (A:B) was 1:1, during which the electrocardiogram was monitored by the Holter electrocardiograph (long-term ECG analyzing system, trade name: HS1000 system, product of FUKUDA M-E KOGYO Co., LTD.) of which electrode was mounted according to standard limb II leads. Results are shown in Fig. 3. Notably, ventricular heart beat was 31 bpm during the monitoring.

After 6 weeks, the pacemaker was turned off to thereby stop applying the electrical stimulation. Atrial fibrillation was confirmed through the electrocardiography in 6 individuals out of 6 individuals of the beagle dogs of Production Example 1, indicating that a model of sustained atrial fibrillation was produced. Also, atrial fibrillation was confirmed to be sustained for 1 week or more in 6 individuals out of 6 individuals.

(Production Example 2)

Beagle dogs (n=4) were subjected to the same treatment as in Production Example 1, except that the period during which the vibration was applied to the atrium at the atrium pacing step was changed from 6 weeks to 4 weeks.

After 4 weeks, the pacemaker was turned off to thereby stop applying the electrical stimulation. Atrial fibrillation was confirmed through the electrocardiography in 4 individuals out of 4 individuals of the beagle dogs of Production Example 2, indicating that the model of sustained atrial fibrillation was produced. However, atrial fibrillation is sustained only for 24 hours to 1 week in 4 individuals out of 4 individuals. Thereafter, atrial fibrillation was spontaneously terminated

and returned to sinus rhythm.

(Comparative Production Example 1)

Beagle dogs (n=6) were subjected to the same treatment as in Production Example 1, except that the pacing speed of the pacemaker at the atrium pacing step was changed from 600 bpm to 400 bpm. During the treatment, the electrocardiogram was monitored by the Holter electrocardiograph (long-term ECG analyzing system, trade name: HS1000 system, product of FUKUDAM-E KOGYO Co., LTD.). Results are shown in Fig. 4. Notably, ventricular heart beat was 35 bpm during the monitoring.
After 6 weeks, the pacemaker was turned off to thereby stop applying the electrical stimulation. Atrial fibrillation was not confirmed through the electrocardiography in 6 individuals out of 6 individuals of the beagle dogs of Comparative Production Example 1. After the electrical stimulation by the pacemaker was stopped, sinus rhythm was recovered. Thus, the model of sustained atrial fibrillation was not produced.

(Comparative Production Example 2)

Beagle dogs (n=4) were subjected to the same treatment as in Production Example 1, except that the position to which the tip electrode of the pacing lead was secured at the atrium pacing step was changed from the Bachmann bundle region in the left atrium to the Bachmann bundle region in the right atrium. Notably, in the case of operating the pacemaker at 600 bpm, the ratio of an electrical stimulation applied by the pacemaker (A) to an atrial muscle contraction reaction (B) (A:B) was 2:1.
After 6 weeks, the pacemaker was turned off to thereby stop applying the electrical stimulation. Atrial fibrillation was not confirmed through the electrocardiography in 4 individuals out of 4 individuals of the beagle dogs of Comparative Production Example 2. After the electrical stimulation by the pacemaker was stopped, sinus rhythm was recovered. Thus, the model of sustained atrial fibrillation was not produced.

(Comparative Production Example 3)

Beagle dogs (n=4) were subjected to the same treatment as in Comparative Production Example 2, except that the pacing speed of the pacemaker at the atrium pacing step was changed from 600 bpm to 400 bpm. Notably, in the case of operating the pacemaker at 400 bpm, the ratio of an electrical stimulation applied by the pacemaker (A) to an atrial muscle contraction reaction (B) (A:B) was 1:1.
After 6 weeks, the pacemaker was turned off to thereby stop applying the electrical stimulation. Atrial fibrillation was not confirmed through the electrocardiography in 4 individuals out of 4 individuals of the beagle dogs of Comparative Production Example 3. After the electrical stimulation by the pacemaker was stopped, sinus rhythm was recovered. Thus, the model of sustained atrial fibrillation was not produced.

(Test Example 1: Screening for atrial fibrillation inhibitor)

Oseltamivir (oseltamivir phosphate, trade name: TAMIFLU (registered trademark), product of CHUGAI PHARMACEUTICAL CO., LTD.; phosphate of the compound represented by Structural Formula (I)), disopyramide (trade name: RYTHMODAN (registered trademark), product of Sanofi K.K.), aprindine (aprindine chloride, trade name: ASPENON (registered trademark), product of Bayer Yakuhin, Ltd), cibenzoline (cibenzoline succinate, trade name: CIBENOL (registered trademark) product of Astellas Pharma Inc.), or pilsicainide (pilsicainide hydrochloride, trade name: SUNRYTHM (registered trademark), product of DAIICHI SANKYO COMPANY, LIMITED), which had been diluted with saline to doses as described in Table 1, was intravenously injected to the model of sustained atrial fibrillation produced in Production Example 1 in doses as described in Table 1 for 10 min. Notably, the doses described in Table 1 are expressed in terms of pure active ingredients.
During from before the administration to after the administration, the electrocardiogram was monitored by the Holter electrocardiograph (long-term ECG analyzing system, trade name: HS1000 system, product of FUKUDA M-E KOGYO Co., LTD.) of which electrode was mounted to the model of sustained atrial fibrillation according to standard limb II leads. After the administration, a frequency of atrial fibrillation termination was determined. Here, the frequency of atrial fibrillation termination is determined according to the following Equation (1). Then, based on the frequency of atrial fibrillation termination, a defibrillation rate was calculated according to the following Equation (2). Both results are shown in Table 1.

$$\text{Frequency of atrial fibrillation termination} = X/Y \cdots \text{Equation (1)}$$

$$\text{Defibrillation rate (\%)} = X/Y \times 100 \cdots \text{Equation (2)}$$

In the Equations (1) and (2), "X" denotes the number of individuals of the model of sustained atrial fibrillation in which atrial fibrillation was terminated after the drug administration, and "Y" denotes the total number of individuals of the model of sustained atrial fibrillation which were subjected to the test.

Table 1

| | Test Substance | Dose (mg/kg) | Administration Time (min) | Frequency of Atrial Fibrillation Termination | Defibrillation Rate (%) |
|---|---|---|---|---|---|
| Oseltamivir-Administered Group | Oseltamivir | 30 | 10 | 11/12 | 91.7 |
| Disopyramide-Administered Group | Disopyramide | 3 | 10 | 1/6 | 16.7 |
| Aprindine-Administered Group | Aprindine | 3 | 10 | 0/6 | 0 |
| Cibenzoline-Administered Group | Cibenzoline | 3 | 10 | 2/6 | 33.3 |
| Pilsicainide-Administered Group | Pilsicainide | 3 | 10 | 2/6 | 33.3 |

As can be seen from Table 1, the frequency of atrial fibrillation termination and the defibrillation rate were very low in the groups to which drugs conventionally used for atrial fibrillation in clinical practice were administered, but the defibrillation rate was very high in the oseltamivir-administered group.

Also, TdP (torsades de pointes: nonsustained polymorphic ventricular tachycardia) was observed in 1 individual out of 6 individuals in the disopyramide-administered group (see Fig. 6A).

Figs. 5A to 5C show electrocardiograms in 1 individual among the oseltamivir-administered group, which is one example in which atrial fibrillation was terminated to return to sinus rhythm due to the drug administration. Figs. 6A to 6C show electrocardiograms in 1 individual among the disopyramide-administered group, which is one example in which atrial fibrillation was not terminated due to the drug administration. In Figs. 5A to 5C, the time point at which the drug administration was started is determined as 0 min, the symbol "-" denotes before the administration and the symbol "+" denotes after the administration on the basis of 0 min.

Fig. 5A shows an electrocardiogram at - 1 min 33 sec before the beginning of administration of oseltamivir, Fig. 5B shows an electrocardiogram at + 2 min 49 sec after the beginning of administration of oseltamivir, and Fig. 5C shows an electrocardiogram at + 5 min 50 sec after the beginning of administration of oseltamivir. From these results, atrial fibrillation was confirmed to be terminated at + 5 min 50 sec after the beginning of administration of oseltamivir.

Fig. 6A shows an electrocardiogram at + 6 min to + 14 min after the beginning of administration of disopyramide, Fig. 6B shows an electrocardiogram at - 22 min 54 sec before the beginning of administration of disopyramide, and Fig. 6C shows an electrocardiogram at + 12 min 15 sec after the beginning of administration of disopyramide and an enlarged view of a portion surrounded by a rectangle in Fig. 6A. From these results, disopyramide could not terminate atrial fibrillation. Further, as can be seen from Fig. 6A, the model of sustained atrial fibrillation in which atrial fibrillation had been occurred and to which disopyramide had been administered was confirmed to be dead.

From the result of Test Example 1, it has been found that oseltamivir is a drug effective for defibrillation, does not adversely affect the ventricle, and has high safety. Therefore, oseltamivir can suitably used as an atrial fibrillation inhibitor.

Also, a method for screening for an atrial fibrillation inhibitor of the present invention can easily and accurately screen new therapeutic drugs for atrial fibrillation, and can suitably used for re-evaluating clinically used therapeutic drugs for atrial fibrillation.

(Test Example 2: Study of dose and combined use of oseltamivir)

Next, a dose of oseltamivir in the case of being used as the atrial fibrillation inhibitor was studied.

Oseltamivir (oseltamivir phosphate, trade name: TAMIFLU (registered trademark), product of CHUGAI PHARMACEUTICAL CO., LTD.; phosphate of the compound represented by Structural Formula (I))) and/or pilsicainide (pilsicainide hydrochloride, trade name: SUNRYTHM (registered trademark), product of DAIICHI SANKYO COMPANY, LIMITED), which had been diluted with saline to doses as described in Table 2-1, was intravenously injected to the model of sustained atrial fibrillation produced in Production Example 1 in doses described in Table 2-1 for 10 min. Notably, the doses described in Table 2-1 are expressed in terms of pure active ingredients.

During from before the administration to after the administration, the electrocardiogram was monitored in the same manner as in Test Example 1, and the frequency of atrial fibrillation termination and the defibrillation rate were determined in the same manner as in Test Example 1. For individuals in which atrial fibrillation was terminated, an electrocardiographic follow-up was performed for 24 hours after atrial fibrillation was terminated to thereby determine whether atrial fibrillation recurred. Results are shown in Tables 2-1 and 2-2.

Table 2-1

| | Test Substance | The number of individuals | Dose (mg/kg) | Administration Time (min) |
|---|---|---|---|---|
| Control group | saline | 4 | - | 10 |
| Low-dose oseltamivir administered group | oseltamivir | 4 | 3 | 10 |
| High-dose oseltamivir administered group | oseltamivir | 5 | 30 | 10 |
| High-dose oseltamivir + pilsicainide administered group | oseltamivir | 2 | 30 | 10 |
| | pilsicainide | | 3 | |

Table 2-2

| | Frequency of Atrial Fibrillation Termination | Defibrillation Rate (%) | Frequency of Atrial Fibrillation Recurrence |
|---|---|---|---|
| Control group | 0/4 | 0 | 0/0 |
| Low-dose oseltamivir administered group | 1/4 | 25 | 0/1 |
| High-dose oseltamivir administered group | 5/5 | 100 | 2/5 |
| High-dose oseltamivir +pilsicainide administered group | 2/2 | 100 | 0/2 |

As can be seen from Tables 2-1 and 2-2, oseltamivir is preferably administered at 30 mg/kg or more. From the result of the high-dose oseltamivir + pilsicainide-administered group, a combination of oseltamivir with pilsicainide was also confirmed to inhibit atrial fibrillation.

Additionally, the high-dose oseltamivir + pilsicainide-administered group was confirmed to have lower frequency of atrial fibrillation recurrence than the high-dose oseltamivir-administered group.

(Test Example 3: Study of effect of oseltamivir on QT prolongation)

Test Example 3 was performed according to the method described in, for example, Hiroshi Yoshida, MD et al., Circ J 2002, Vol. 66, 857-862, A. Sugiyama et al., European Journal of Pharmacology, 2003, Vol. 466, 137-146.

Specifically, normal beagle dogs (about one year old, body weight: about 10 kg, female, KITAYAMA LABES CO., LTD.) were intravenously injected with 30 mg/kg of pentobarbital (product of Mitsubishi Tanabe Pharma Corporation), and simultaneously artificially ventilated by inserting a ventilator (SN-480-3, product of Shinano) into the trachea to thereby supply 20 mL/kg of oxygen. Then, a catheter electrode for pacing (catalog number: 1675P, product of EP Technologies, Inc.) was inserted through the femoral vein into the right ventricle, and then, a tip electrode of the catheter was sewn on and secured to the endocardium of the intraventricular septum within the right ventricle.

Oseltamivir (oseltamivir phosphate, trade name: TAMIFLU (registered trademark), product of CHUGAI PHARMACEUTICAL CO., LTD.; phosphate of the compound represented by Structural Formula (I))), pilsicainide (pilsicainide hydrochloride, trade name: SUNRYTHM (registered trademark), product of DAIICHI SANKYO COMPANY, LIMITED), disopyramide(trade name: RYTHMODAN (registered trademark), product of Sanofi K.K.), or mexiletine (mexiletine hydrochloride, trade name: MEXITIL (registered trademark), product of Nippon Boehringer Ingelheim Co., Ltd.), which had been diluted with saline to doses as described in Table 3, was intravenously injected to the beagle dogs in doses and administration times as described in Table 3. Notably, the above-listed drugs were administered according to the administration schedules described in Table 3. That is, the above-listed drugs were first administered in the lowest concentrations described in Table 3 to the beagle dogs. Then, the concentrations were increased stepwise every 30 min as described in Table 3. Notably, the doses described in Table 3 are expressed in terms of pure active ingredients.

In addition, groups to which saline was administered in the same manner as in the above-listed drugs in terms of the administration time and the administration schedule were used as control groups relative to the above-listed drug-administered groups.

Table 3

| Test Substance | Test Substance | The Number of Individuals | Dose (mg/kg) | Administration Time | |
|---|---|---|---|---|---|
| | | | | Total (min) | Administration Schedule |
| Oseltamivir-Administered Group | Oseltamivir | 4 | 0.3 | 10 | 0 min to 10 min |
| | | | 3 | 10 | 30 min to 40 min |
| | | | 30 | 10 | 60 min to 70 min |
| Pilsicainide-Administered Group | Pilsicainide | 4 | 1 | 10 | 0 min to 10 min |
| | | | 3 | 10 | 30 min to 40 min |
| Disopyramide-Administered Group | Disopyramide | 6 | 0.3 | 10 | 0 min to 10 min |
| | | | 3 | 10 | 30 min to 40 min |
| Mexiletine-Administered Group | Mexiletine | 6 | 0.3 | 0.5 | 0 min to 0.5 min |
| | | | 3 | 0.5 | 30 min to 30.5 min |

During from before the administration to after the administration, QT interval (ms), PR interval (ms), P wave (ms), QRS wave (ms), $MAP_{90\ (sinus)}$, AH interval, and HV interval during sinus rhythm were measured by the Holter electrocardiograph (long-term ECG analyzing system, trade name: HS1000 system, product of FUKUDA M-E KOGYO Co., LTD.) of which electrode was mounted to the model of sustained atrial fibrillation according to standard limb II leads.

Notably, P wave (P duration) indicates an atrial electrical excitation, QRS wave indicates a ventricular electrical excitation, AH denotes atrio-His interval, HV denotes His-ventricular interval, and $MAP_{90\ (sinus)}$ denotes the time for which the action potential amplitude is repolarized to 90% of the maximum value thereof during sinus rhythm.

Also, MAP (Monophasic action potential) was amplified with a DC pre-amplifier (catalog number: 300, product of EP Technologies, Inc.), and measured. For pacing the heart, a cardiac stimulator (catalog number: SEC-3102, product of NIHON KOHDEN CORPORATION) was used to stimulate the ventricle at 1 V to 2 V (about twice the stimulus threshold).

At a cycle length of 300 msec, the pacing of the ventricle was performed and $MAP_{90\ (CL300)}$ was measured. At a cycle length of 400 msec, the pacing of the ventricle was performed and $MAP_{90\ (CL400)}$ was measured.

The ventricle was subjected to the pacing 8 times at the cycle length of 400 msec, followed by applying a shorter

stimulus at the 9th time. The coupling interval of the shorter stimulus was shortened with a decrement of 5 ms to 10 ms to thereby determine ERP (Effective refractory period) $_{(CL400)}$. As used herein, the term "shorter stimulus" refers to a shorter cycle length than 400 msec. Also, "coupling interval of shorter stimulus" refers to an interval between the time point of the 8th pacing (i.e., the last stimulus at the cycle length of 400 msec) and the time point of the 9th stimulus (i.e., the shorter stimulus).

Notably, the ventricular heart rate was 200 bpm in the case where the ventricle was subjected to the pacing at the cycle length of 300 msec, whereas the ventricular heart rate was 150 bpm in the case the ventricle was subjected to the pacing at the cycle length of 400 msec.

Also, QTc (corrected QT interval) (ms) was calculated according to the following Bazett's Formula (1920), and TRP (Torsades de Points: terminal repolarization period) (ms) was calculated according to the following equation.

$$QTc = QT / \sqrt{RR} \cdots \text{Bazett's Formula}$$

$$TRP = MAP_{90\,(CL400)} - ERP$$

Fig. 7 shows results of the oseltamivir-administered group, Fig. 8 shows results of the pilsicainide-administered group, Fig. 9 shows results of the disopyramide-administered group, and Fig. 10 shows results of the mexiletine-administered group. In Figs. 7 to 10, black circles, black squares, and black triangles denote being significantly different by $p < 0.05$.

As can be seen from Figs. 7 to 10, the oseltamivir-administered group showed short prolongations of QT and QTc and a safe QT prolongation pattern, suggesting that oseltamivir can be administered at high dose. Meanwhile, the pilsicainide-administered group, the disopyramide-administered group, and the mexiletine-administered group showed longer prolongations of QT and QTc than that of the oseltamivir-administered group, and a dangerous QT prolongation pattern. The QT interval is an index for estimating the action potential duration of myocardial cells. Excessive prolongation of the QT interval has been known to provoke TdP (Torsades de Pointes) which is fatal ventricular arrhythmia. The longer the QT interval prolongation is, the higher the risk of developing TdP is.

Also, the oseltamivir-administered group had greater $MAP_{90\,(CL300)}$, which indicates a prolongation at the heart rate of 200 bpm, than $MAP_{90\,(CL400)}$, which indicates a prolongation at the heart rate of 150 bpm, and showed a safe MAP prolongation pattern. Meanwhile, the pilsicainide-administered group and the disopyramide-administered group had greater $MAP_{90\,(CL400)}$, which indicates a prolongation at the heart rate of 150 bpm, than $MAP_{90\,(CL300)}$, which indicates a prolongation at the heart rate of 200 bpm, and showed a dangerous MAP prolongation pattern.

Additionally, the oseltamivir-administered group showed a longer prolongation of P wave, suggesting high selectivity for the atrium. The oseltamivir-administered group showed a longer prolongation of P wave width on the electrocardiogram, which is an index reflecting an inhibitory effect on $Na^+$ channels in the atrial muscle, than indices of those of on other ion channels (see an upper left section in Fig. 7).

From these results, it has been found that oseltamivir is more excellent in inhibitory effect on atrial fibrillation than drugs conventionally used in clinical practice as preventive drugs or therapeutic drugs for arrhythmia including atrial fibrillation; and has high safety with reduced adverse side effects.

Industrial Applicability

An atrial fibrillation inhibitor and an antiarrhythmic drug of the present invention have an excellent inhibitory effect on arrhythmia including atrial fibrillation and have high safety with reduced adverse side effects, so that they can be suitably used as a preventive drug or a therapeutic drug for arrhythmia including atrial fibrillation. Further, the atrial fibrillation inhibitor and the antiarrhythmic drug are effective for sustained atrial fibrillation, so that they can prevent progression to permanent atrial fibrillation. Additionally, they can be suitably used as a preventive drug for complicating diseases of atrial fibrillation such as thrombotic or embolic diseases of organs in the whole body (e.g., cerebral infarction).

The model of sustained atrial fibrillation of the present invention is a model which simulates well clinical sustained atrial fibrillation, which allows atrial fibrillation to be sustained, and in which atrial fibrillation can be inhibited or defibrillated by using drugs. Therefore, the model can be suitably used for screening for atrial fibrillation inhibitors.

The method for screening for atrial fibrillation inhibitors of the present invention is excellent as an in vivo evaluation system of atrial fibrillation because a canine model of sustained atrial fibrillation is used which has a similar developmental mechanism to human atrial fibrillation. In addition, the method can effectively and efficiently used for screening for new atrial fibrillation inhibitors used for preventing or treating atrial fibrillation; or re-evaluating existing therapeutic drugs for atrial fibrillation.

## Claims

1. An atrial fibrillation inhibitor, comprising:

a compound expressed by one of the following Structural Formulas (I) to (VI) or a pharmacologically acceptable salt thereof,
wherein the atrial fibrillation inhibitor inhibits atrial fibrillation:

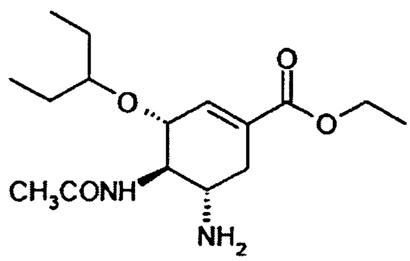

Structural Formula (I)

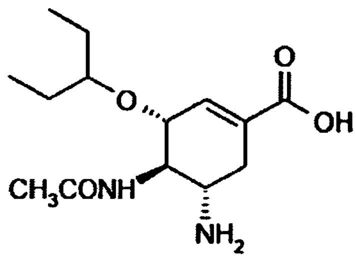

Structural Formula (II)

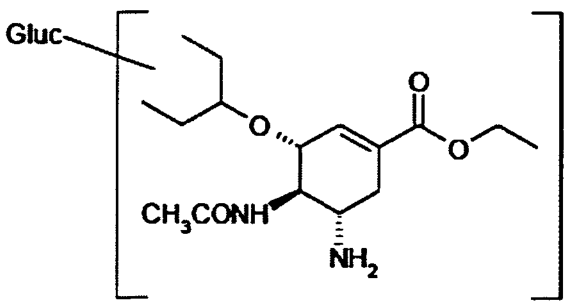

Structural Formula (III)

where in the Structural Formula (III), Gluc refers to glucuronic acid,

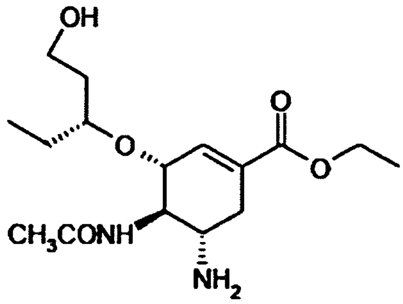

Structural Formula (IV)

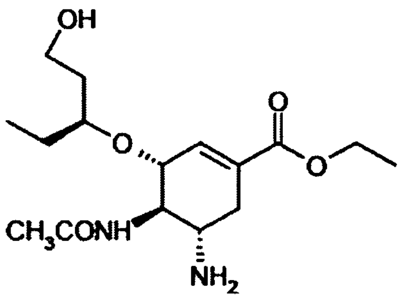

Structural Formula (V)

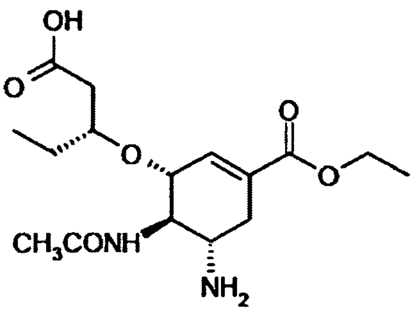

Structural Formula (VI).

**2.** The atrial fibrillation inhibitor according to claim 1, wherein the atrial fibrillation is idiopathic atrial fibrillation or chronic atrial fibrillation.

**3.** A method for producing a model of sustained atrial fibrillation, comprising:

inserting an electrode catheter into a region of a dog containing an atrioventricular node and applying high-frequency current to the region from the electrode catheter to break the atrioventricular node and block atrioventricular conduction, to thereby dilate and enlarge the atrium thereof; and
pacing the atrium which has been dilated and enlarged.

**4.** The method for producing a model of sustained atrial fibrillation according to claim 3, wherein the pacing is pacing the atrium at 500 bpm to 700 bpm for 4 weeks or more.

**5.** A model of sustained atrial fibrillation, wherein the model of sustained atrial fibrillation is produced by the method for producing a model of sustained atrial fibrillation according to claim 3 or 4.

6. The model of sustained atrial fibrillation according to claim 5, wherein atrial fibrillation is sustained for at least one week.

7. A method for screening for an atrial fibrillation inhibitor, comprising:

administering a test substance to the model of sustained atrial fibrillation according to claim 5 or 6; and screening for, as an atrial fibrillation inhibitor, the test substance that inhibits atrial fibrillation in the model of sustained atrial fibrillation after the administering.

8. The method for screening for an atrial fibrillation inhibitor according to claim 7, further comprising: pacing the atrium of the model of sustained atrial fibrillation in which atrial fibrillation has been inhibited in the screening, to thereby re-induce atrial fibrillation therein.

9. The method for screening for an atrial fibrillation inhibitor according to claim 8, wherein the pacing is pacing the atrium of the model of sustained atrial fibrillation at 500 bpm to 700 bpm for at least 1 week within 1 week after atrial fibrillation has been inhibited.

10. An antiarrhythmic drug, comprising:

a compound expressed by one of the following Structural Formulas (I) to (VI) or a pharmacologically acceptable salt thereof,
wherein the antiarrhythmic drug inhibits arrhythmia:

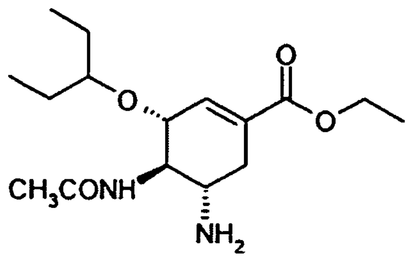

Structural Formula (I)

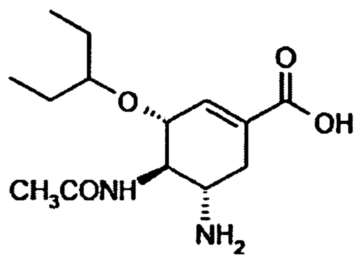

Structural Formula (II)

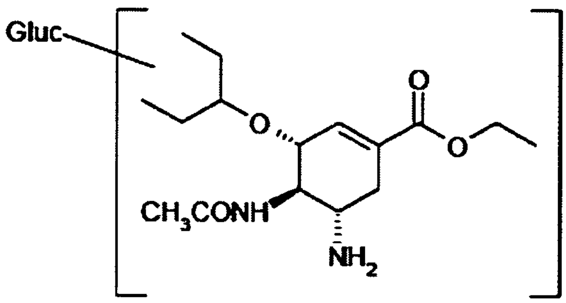

Structural Formula (III)

where in the Structural Formula (III), Gluc refers to glucuronic acid,

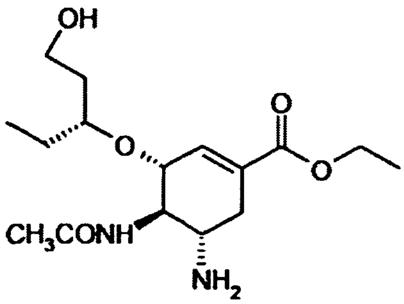

Structural Formula (IV)

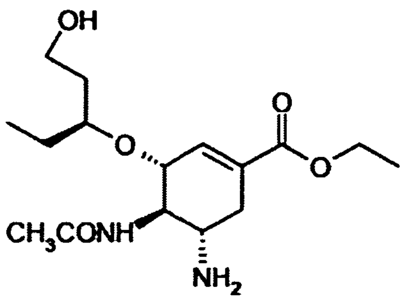

Structural Formula (V)

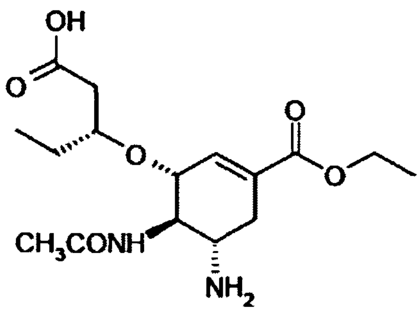

Structural Formula (VI).

FIG. 1A

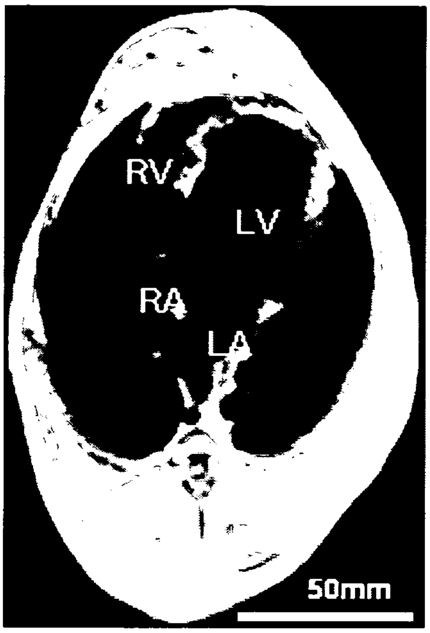


FIG. 1B

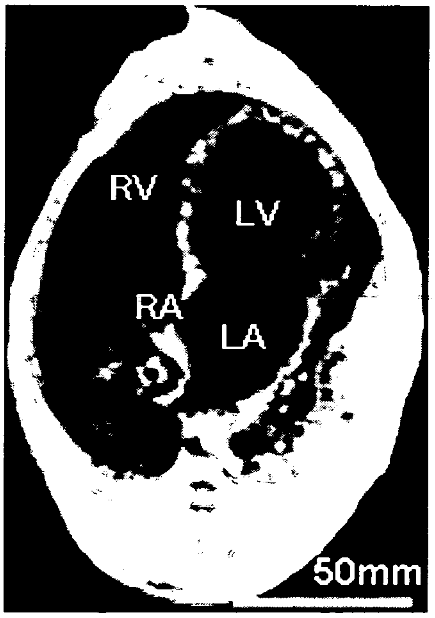

FIG. 2A

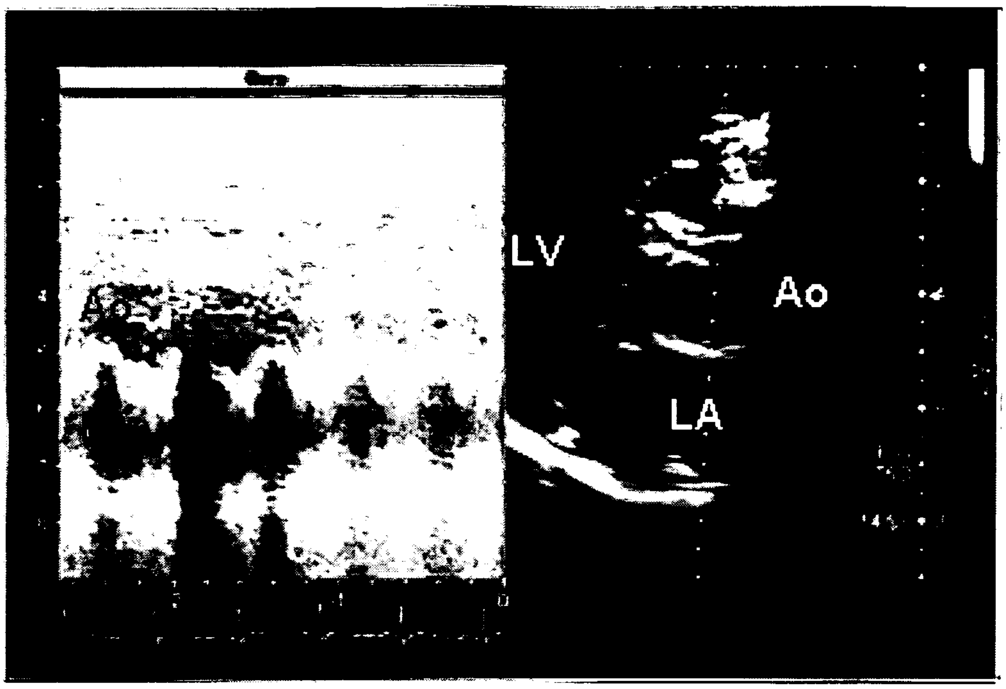

FIG. 2B

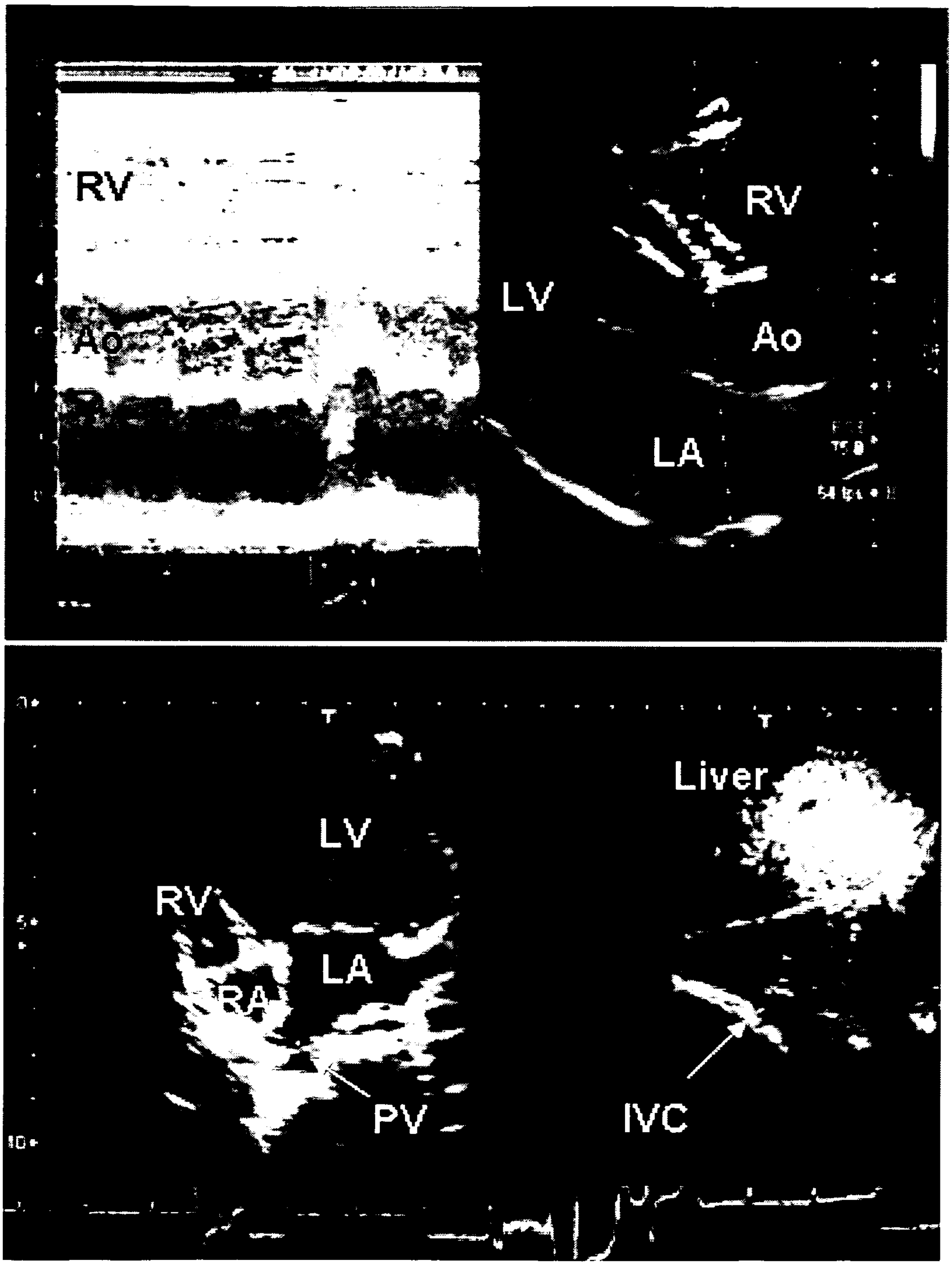

FIG. 2C

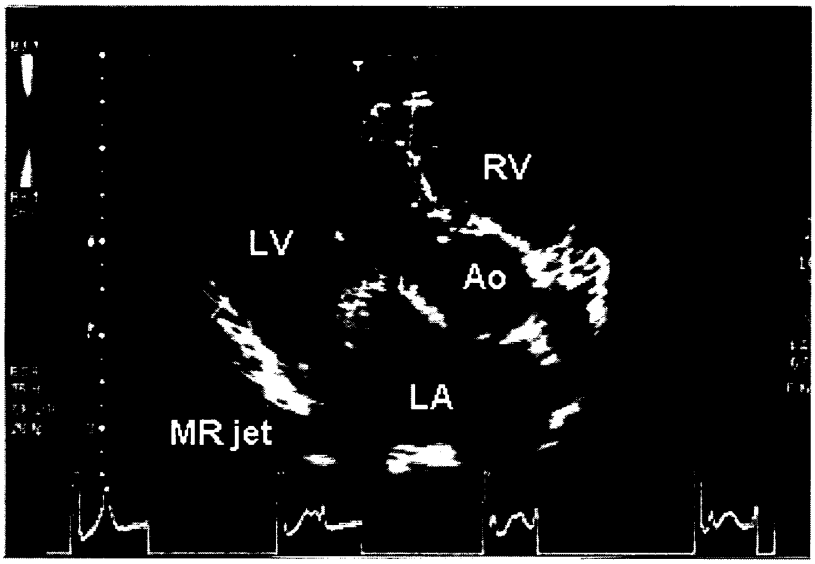


FIG. 3

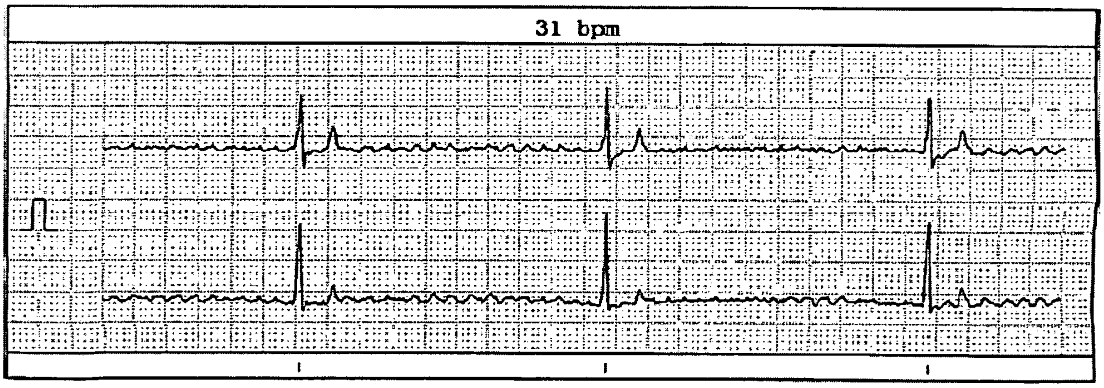

FIG. 4

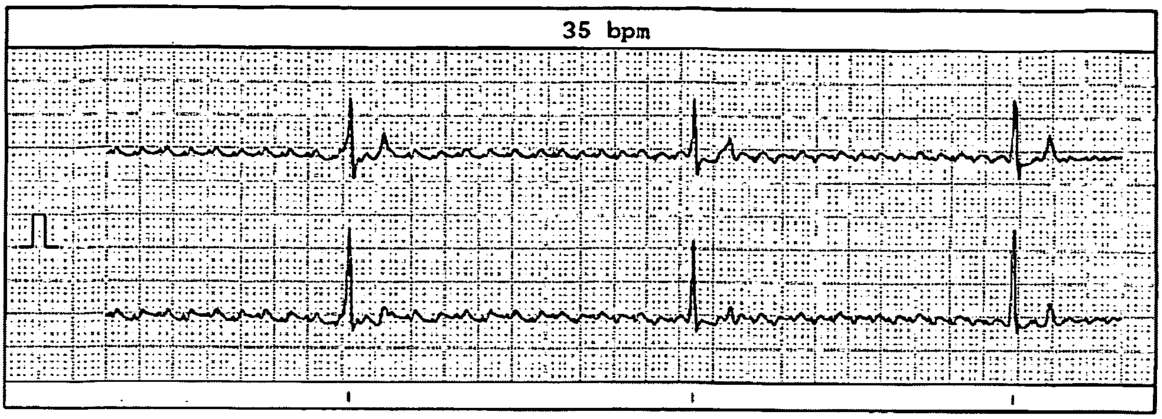


FIG. 5A

– 1 min 33 sec (before the beginning of administration)

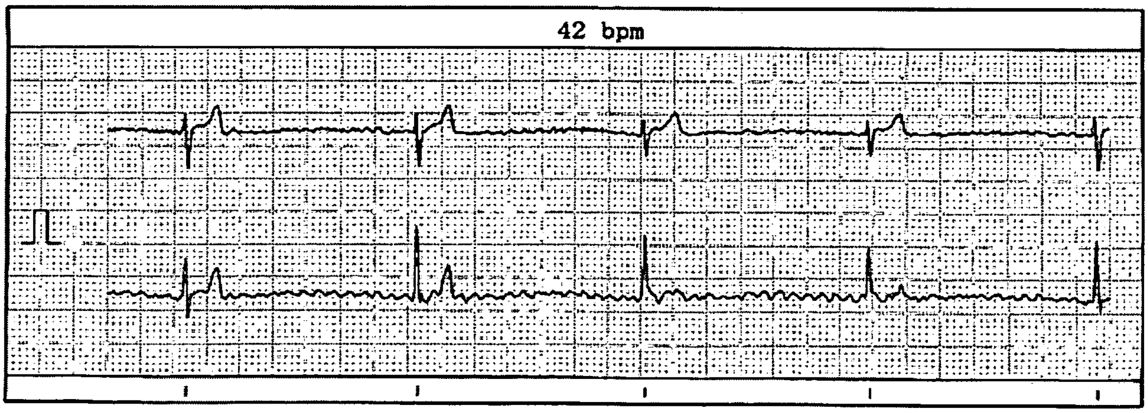

FIG. 5B

+ 2 min 49 sec (after the beginning of administration)

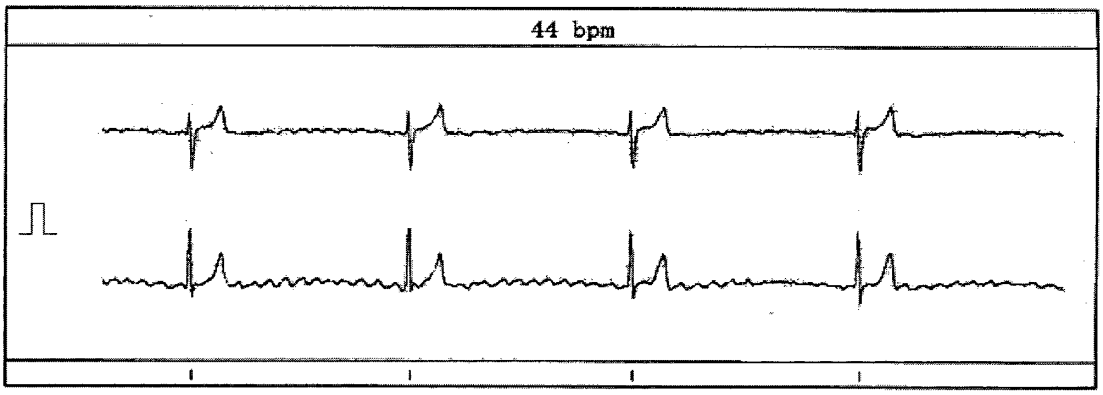


FIG. 5C

+ 5 min 50 sec (after the beginning of administration)

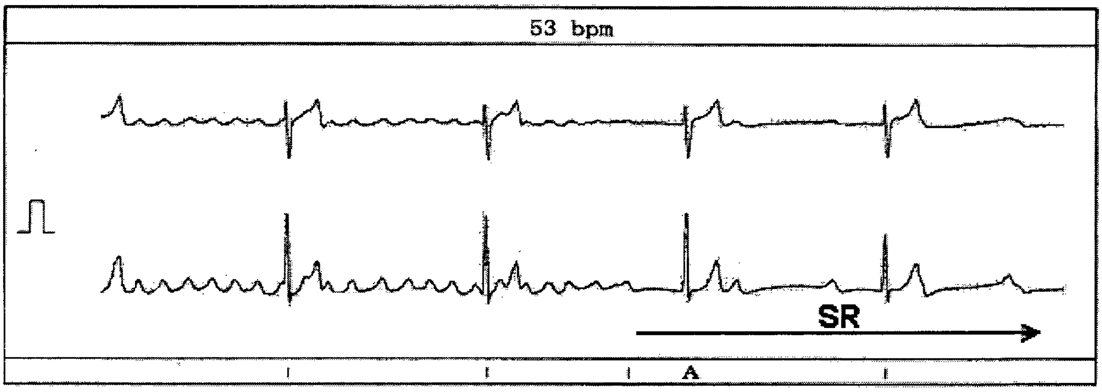

# FIG. 6A

+ 6 min 00 sec to + 14 min 00 sec

(after the beginning of administration)

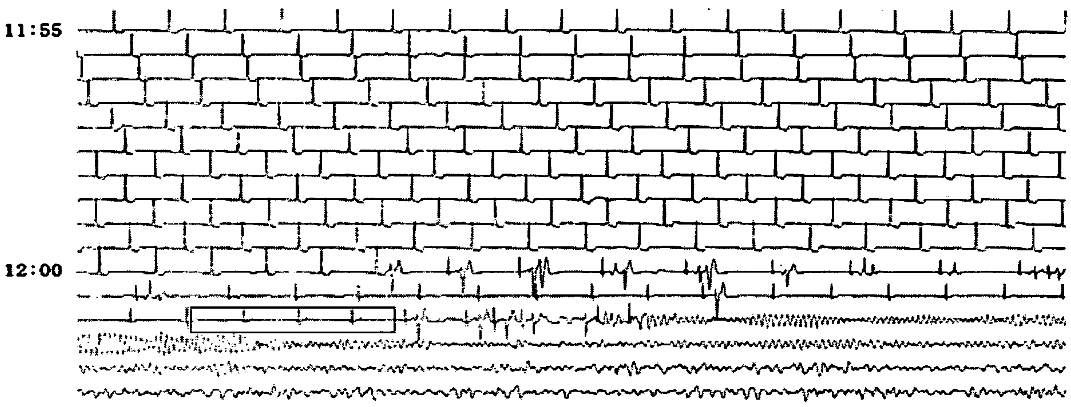

FIG. 6B

– 22 min 54 sec (before the beginning of administration)

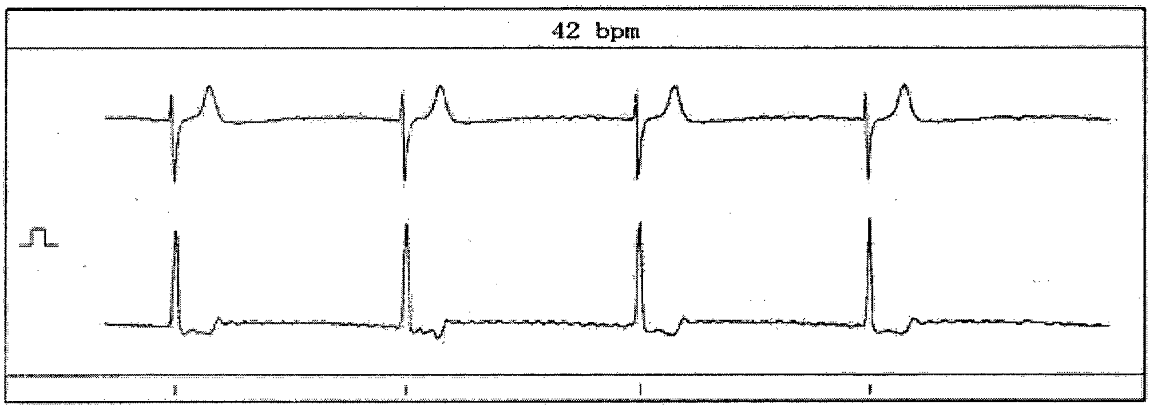


FIG. 6C

+ 12 min 15 sec (after the beginning of administration)

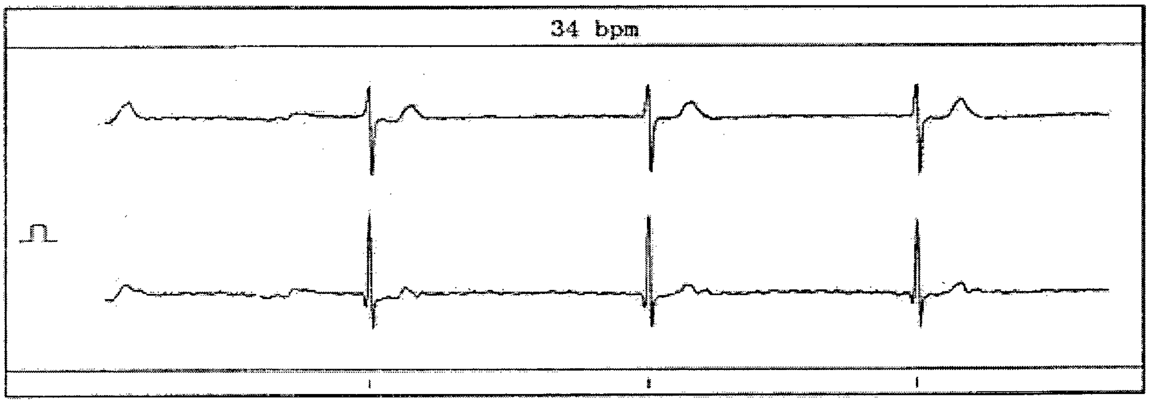

# FIG. 7

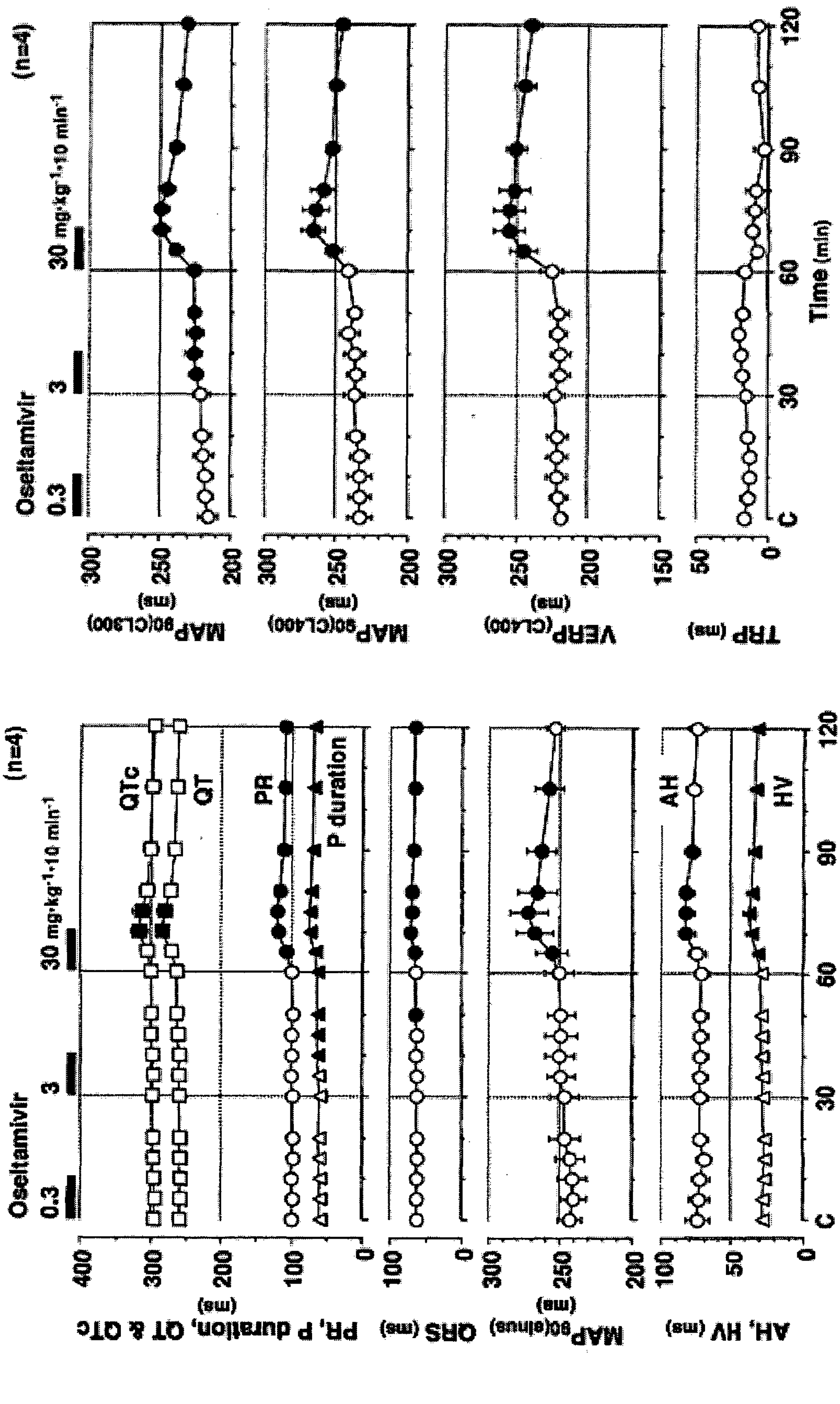
Oseltamivir
0.3
3
30 mg·kg-1·10 min-1
(n=4)
QTc
QT
PR
P duration
AH
HV
PR, P duration, QT & QTc (ms)
QRS (ms)
MAP90(sinus) (ms)
AH, HV (ms)
MAP90(CL300) (ms)
MAP90(CL400) (ms)
VERP(CL400) (ms)
TRP (ms)
Time (min)

FIG. 8

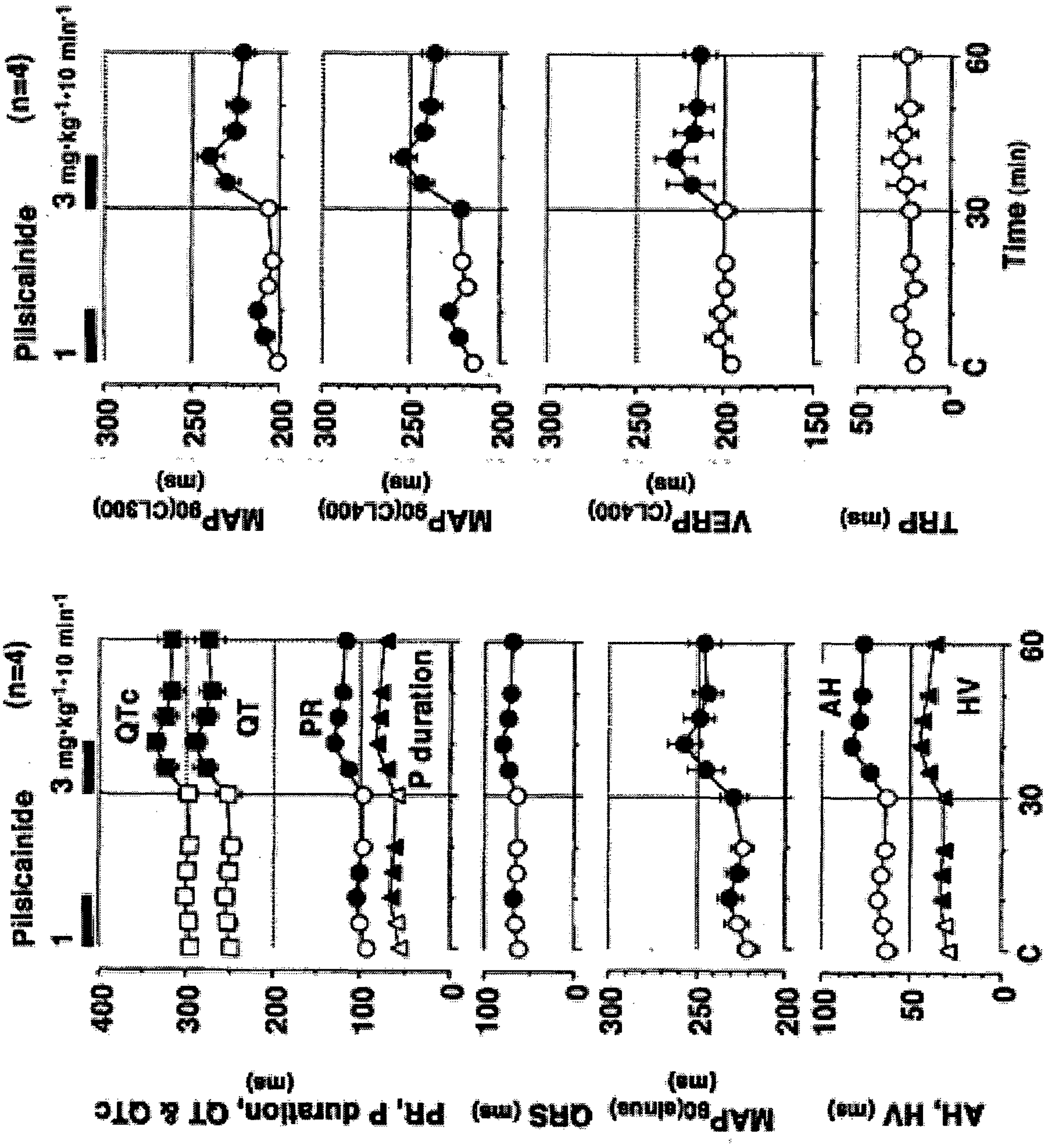
Pilsicainide
1
3 mg·kg⁻¹·10 min⁻¹
(n=4)
MAP90(CL300) (ms)
MAP90(CL400) (ms)
VERP(CL400) (ms)
TRP (ms)
PR, P duration, QT & QTc (ms)
QTc
QT
PR
P duration
QRS (ms)
MAP90(sinus) (ms)
AH, HV (ms)
AH
HV
Time (min)
C
30
60

FIG. 9

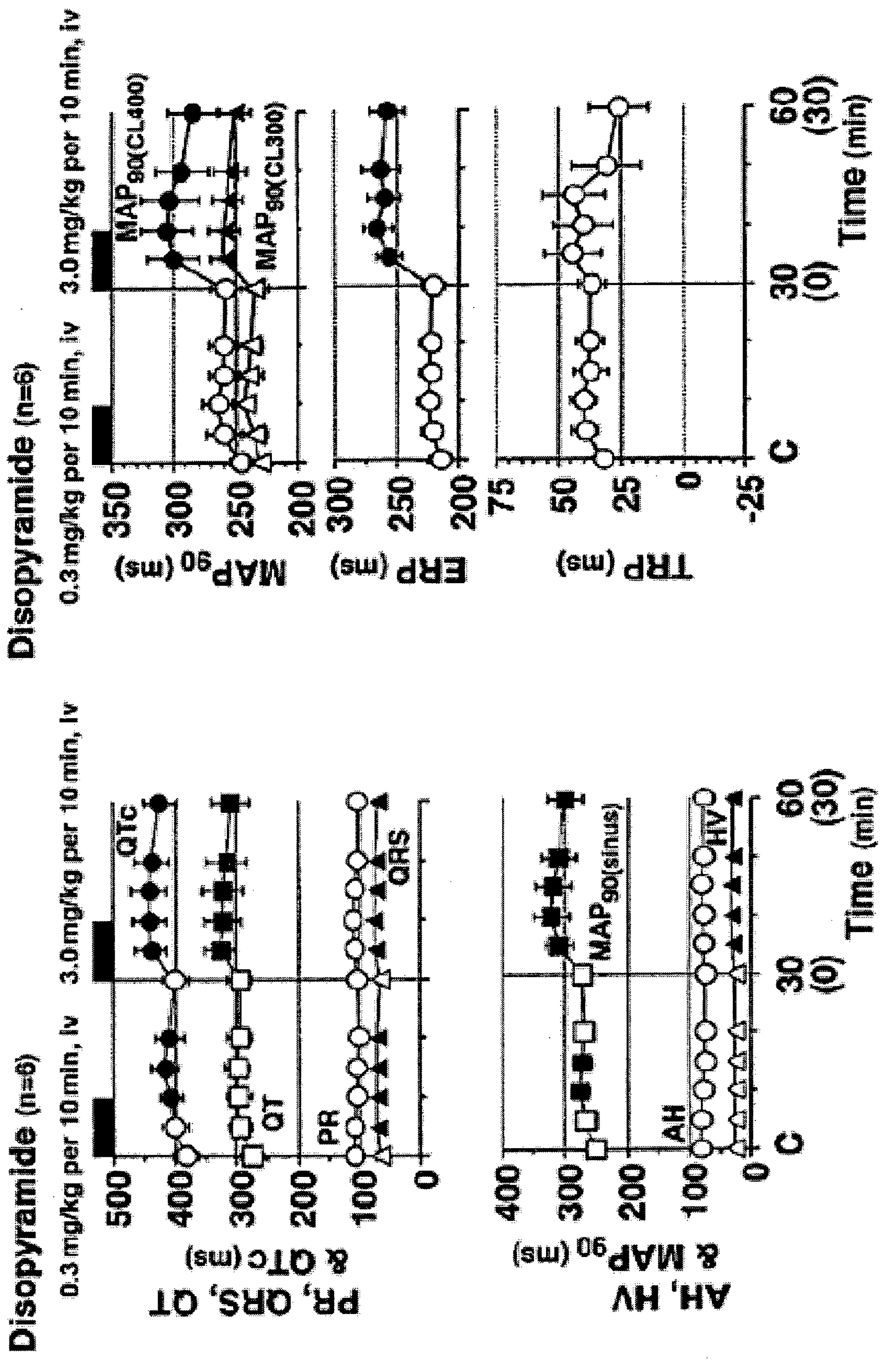
Disopyramide (n=6)
0.3 mg/kg per 10 min, iv
3.0 mg/kg per 10 min, iv
PR, QRS, QT & QTc (ms)
QTc
QT
PR
QRS
AH, HV & MAP90 (ms)
MAP90(sinus)
AH
HV
MAP90 (ms)
MAP90(CL400)
MAP90(CL300)
ERP (ms)
TRP (ms)
C
30 (0)
60 (30)
Time (min)

FIG. 10

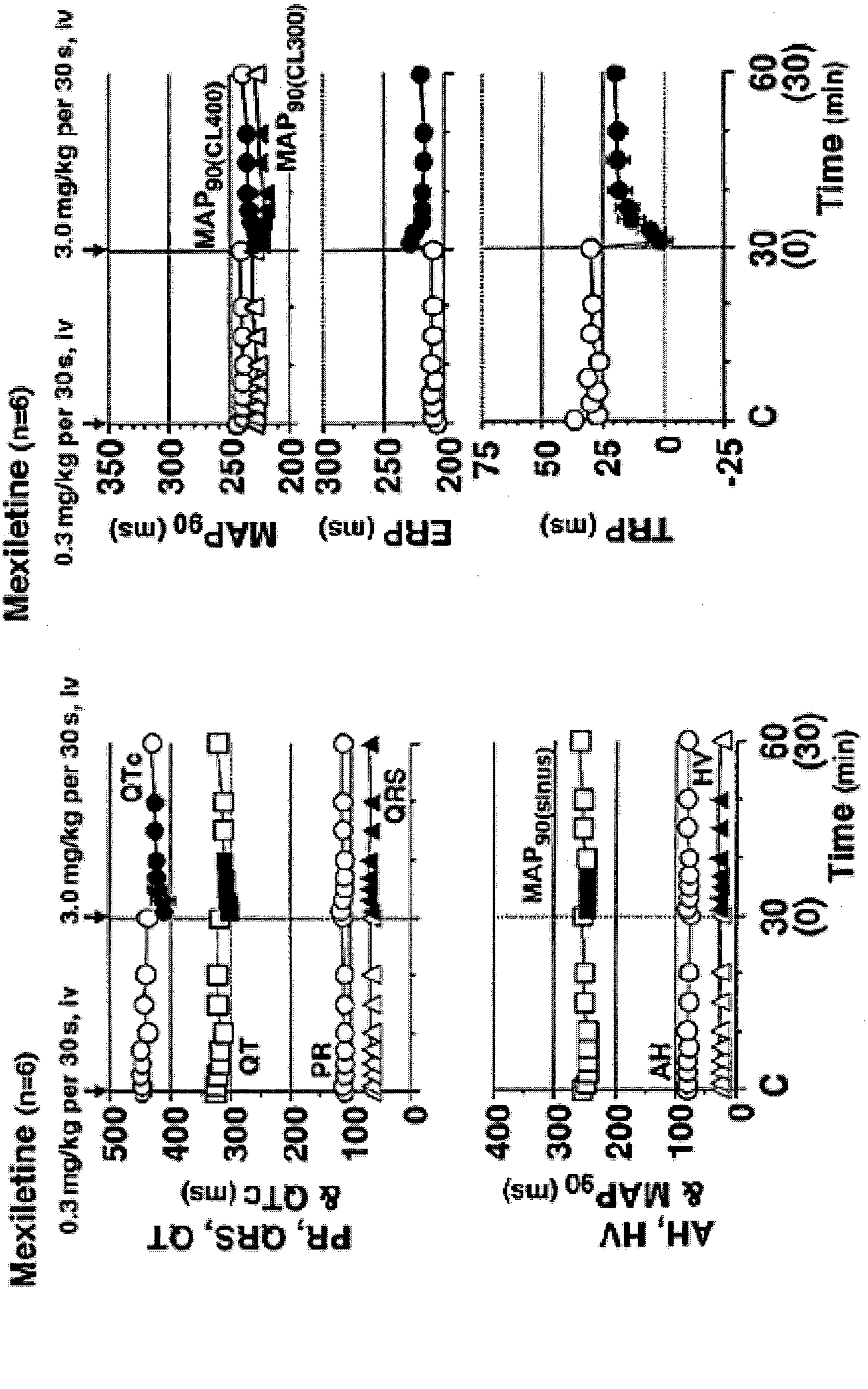
Mexiletine (n=6)
0.3 mg/kg per 30 s, iv
3.0 mg/kg per 30 s, iv
MAP90 (ms)
MAP90(CL400)
MAP90(CL300)
ERP (ms)
TRP (ms)
Time (min)
PR, QRS, QT & QTc (ms)
QTc
QT
PR
QRS
AH, HV & MAP90 (ms)
MAP90(sinus)
AH
HV

**INTERNATIONAL SEARCH REPORT**

International application No.
PCT/JP2012/061314

A. CLASSIFICATION OF SUBJECT MATTER
*A61K31/215*(2006.01)i, *A01K67/027*(2006.01)i, *A61K31/231*(2006.01)i, *A61P9/06*(2006.01)i, *G01N33/15*(2006.01)i, *G01N33/50*(2006.01)i

According to International Patent Classification (IPC) or to both national classification and IPC

B. FIELDS SEARCHED

Minimum documentation searched (classification system followed by classification symbols)
A61K31/215, A01K67/027, A61K31/231, A61P9/06, G01N33/15, G01N33/50

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
CAPlus/REGISTRY/MEDLINE/EMBASE/BIOSIS(STN), JSTPlus(JDreamII), JMEDPlus(JDreamII), JST7580(JDreamII)

C. DOCUMENTS CONSIDERED TO BE RELEVANT

| Category* | Citation of document, with indication, where appropriate, of the relevant passages | Relevant to claim No. |
|---|---|---|
| A | ZHANG,X. et al, Clinical investigation of the clinical management and therapeutic stretagies to the first human case infected by influenza A/H5N1 in Guizhou Province, Chinese Journal of Tuberculosis and Respiratory Diseases, 2009, Vol.32, No.5, p.342-346, entire text, particularly, Abstract | 1,2,10 |
| A | Kotaro TAKAMATSU et al., "Oseltamivir Phosphate Heiyo de Warfarin Koka ga Genjaku shita Ikkasei Nokyoketsu Hossa no Nirei", Clinical Neurology, vol.46, no.2, 2006, page 177, entire text | 1,2,10 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 05 July, 2012 (05.07.12) | 17 July, 2012 (17.07.12) |
| Name and mailing address of the ISA/ Japanese Patent Office | Authorized officer |
| Facsimile No. | Telephone No. |

Form PCT/ISA/210 (second sheet) (July 2009)

**INTERNATIONAL SEARCH REPORT**

International application No.
PCT/JP2012/061314

C (Continuation). DOCUMENTS CONSIDERED TO BE RELEVANT

| Category* | Citation of document, with indication, where appropriate, of the relevant passages | Relevant to claim No. |
|---|---|---|
| A | WELLS,Q. et al, Sotalol-induced torsades de pointes precipitated during treatment with oseltamivir for H1N1 influenza, Heart Rhythm, 2010, Vol.7, No.10, p.1454-7, entire text, particularly, Abstract | 1,2,10 |
| P,X | Sanae SUZUKI et al., "Marmot Shinbo ni Okeru oseltamivir no Denki Seiri Sayo Tokusei", Jisedai o Ninau Soyaku Iryo Yakuri Symposium Program Yoshishu, 2011 Aug, vol.2011, page 76, entire text | 1,2,10 |
| P,A | NAKAMURA,Y et al, Oseltamivir lacks torsadogenic potential, J Pharmacol Sci, Vol.118, No.Supplement 1, 2012 Feb, p.88P, entire text | 1,2,10 |
| P,A | KITAHARA,K et al, In vivo electropharmacological effects of oseltamivir, J Pharmacol Sci, Vol.118, No.Supplement 1, 2012 Feb, p.88P, entire text | 1,2,10 |

Form PCT/ISA/210 (continuation of second sheet) (July 2009)

**INTERNATIONAL SEARCH REPORT**

International application No.
PCT/JP2012/061314

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet.

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.:
Claims 1, 2 and 10

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

INTERNATIONAL SEARCH REPORT

International application No.
PCT/JP2012/061314

Continuation of Box No.III of continuation of first sheet(2)

"A method for producing a model of sustained atrial fibrillation, comprising inserting an electrode catheter into a canine atrioventricular nodal region - - -" according to the invention in claim 3 of the present application does not correspond to a method that is particularly applied to produce "an atrial fibrillation inhibitor characterized by comprising a compound represented by any of structural formulae (I) to (VI) - - - and inhibiting atrial fibrillation" according to the invention in claim 1 of the present application.

Thus, these inventions have a common technical feature exclusively in "atrial fibrillation".

Needless to say, however, the aforesaid disease has been known in public. Thus, this common technical feature does not make a contribution over the prior art and, therefore, cannot be considered as a special technical feature.

Further, there is no other same or corresponding special technical feature between these inventions.

Accordingly, the following two inventions are involved in claims.

(Invention 1) the inventions of claims 1, 2 and 10 of the present application

An atrial fibrillation inhibitor and an antiarrhythmic drug characterized by comprising a compound represented by any of structural formulae (I) to (VI) - - - and inhibiting atrial fibrillation.

(Invention 2) the inventions of claims 3-9 of the present application

A method for producing a model of sustained atrial fibrillation and a method for screening an atrial fibrillation inhibitor using the aforesaid model.

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2000517306 A
- JP 2006067950 A


### Non-patent literature cited in the description


- **MOE et al.** *Am. Heart. J.,* 1959, vol. 58, 59-70
- **PAGE et al.** *J. Am Coll Cardiol.,* 1986, vol. 8, 872-879
- **MAURITS C.E.F. WIJFFELS et al.** *Circulation,* 1995, vol. 92, 1954-1968
- **MARCH.** Advanced Organic Chemistry. 425-427
- **LAROCK.** *Comprehensive Organic Transformations,* 604-614
- **GREENE.** *Protective Groups in Organic Synthesis,* 218-287
- Merck Index. 8071
- Pharmaceutical Interview Form. December 2005
- **HIROSHI YOSHIDA, MD et al.** *Circ J,* 2002, vol. 66, 857-862
- **A. SUGIYAMA et al.** *European Journal of Pharmacology,* 2003, vol. 466, 137-146